(19) Europäisches Patentamt / European Patent Office / Office européen des brevets

(11) **EP 3 411 689 B1**

(12) **EUROPEAN PATENT SPECIFICATION**

(45) Date of publication and mention of the grant of the patent:
**06.07.2022 Bulletin 2022/27**

(21) Application number: **16889601.7**

(22) Date of filing: **04.02.2016**

(51) International Patent Classification (IPC):
G01N 21/01 (2006.01)  G01N 21/17 (2006.01)
G01N 15/05 (2006.01)  A61B 5/145 (2006.01)
G01J 3/02 (2006.01)  G01J 3/10 (2006.01)
G01J 3/14 (2006.01)  G01J 3/42 (2006.01)
G01N 21/03 (2006.01)  G01N 21/27 (2006.01)
G01N 21/31 (2006.01)  G01N 21/49 (2006.01)
G01N 21/21 (2006.01)  G01N 33/49 (2006.01)

(52) Cooperative Patent Classification (CPC):
G01N 21/274; A61B 5/14535; G01J 3/0205;
G01J 3/0208; G01J 3/0291; G01J 3/10; G01J 3/14;
G01J 3/42; G01N 21/0303; G01N 21/31;
G01N 33/492; G01N 2021/216; G01N 2201/062;
G01N 2201/0634

(86) International application number:
**PCT/US2016/016560**

(87) International publication number:
**WO 2017/135952 (10.08.2017 Gazette 2017/32)**

(54) **SYSTEM AND METHOD FOR OPTICAL ABSORBANCE MEASUREMENT OF WHOLE BLOOD**

SYSTEM UND VERFAHREN ZUR OPTISCHEN EXTINKTIONSMESSUNG VON VOLLBLUT

SYSTÈME ET PROCÉDÉ DE MESURE D'ABSORPTION OPTIQUE DE SANG TOTAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.12.2018 Bulletin 2018/50**

(60) Divisional application:
**22174592.0**
**22174601.9**
**22174613.4**
**22174620.9**

(73) Proprietor: **Nova Biomedical Corporation**
**Waltham, MA 02454 (US)**

(72) Inventors:
• **CAFFERTY, Michael**
**Medford, Massachusetts 02155 (US)**
• **CIONEK, Scott P.**
**Bolton, Massachusetts 01740 (US)**

(74) Representative: **Katérle, Axel**
**Wuesthoff & Wuesthoff**
**Patentanwälte PartG mbB**
**Schweigerstraße 2**
**81541 München (DE)**

(56) References cited:
WO-A1-2015/076262    WO-A2-2012/116336
CN-C- 1 268 916    JP-A- S5 928 642
JP-A- 2009 053 029    US-A- 1 143 675
US-A- 2 850 941    US-A- 3 569 721
US-A- 4 310 249    US-A- 5 934 278
US-A- 6 049 727    US-A1- 2005 037 505
US-A1- 2005 094 127    US-A1- 2005 259 254
US-A1- 2007 060 810    US-A1- 2008 274 554
US-A1- 2009 079 965    US-A1- 2015 021 480
US-A1- 2015 316 471    US-B1- 6 235 241
US-B1- 6 650 412

**Description**

**BACKGROUND OF THE INVENTION**

1. Field of the Invention

The present invention relates generally to spectroscopic systems and methods for the identification and characterization of hemoglobin parameters in blood.

2. Description of the Prior Art

An ultraviolet-visible light spectroscopic system involves absorption spectroscopy or reflectance spectroscopy. As the name implies, such systems use light in the visible and near ultraviolet ranges for analyzing a sample. The wavelength range is typically from about 400 nm to about 700 nm. The absorption or reflectance of the visible light directly affects the perceived color of the chemicals involved. UV/Vis spectroscopy is routinely used in analytical chemistry for the quantitative determination of different analytes, such as transition metal ions, highly conjugated organic compounds, and biological macromolecules. Spectroscopic analysis is commonly carried out in solutions but solids and gases may also be studied.

A near-infrared spectroscopic system also involves absorption spectroscopy or reflectance spectroscopy. Such systems use light in the near-infrared range for analyzing a sample. The wavelength range is typically from about 700 nm to less than 2,500 nm. Typical applications include pharmaceutical, medical diagnostics (including blood sugar and pulse oximetry), food and agrochemical quality control, and combustion research, as well as research in functional neuroimaging, sports medicine & science, elite sports training, ergonomics, rehabilitation, neonatal research, brain computer interface, urology (bladder contraction), and neurology (neurovascular coupling).

Instrumentation for near-IR (NIR) spectroscopy is similar to instruments for the UV-visible and mid-IR ranges. The basic parts of a spectrophotometer are a light source, a holder for the sample, a diffraction grating in a monochromator or a prism to separate the different wavelengths of light, and a detector. The radiation source is often a Tungsten filament (300-2500 nm), a deuterium arc lamp, which is continuous over the ultraviolet region (190-400 nm), Xenon arc lamp, which is continuous from 160-2,000 nm, or more recently, light emitting diodes (LED) for the visible wavelengths. The detector is typically a photomultiplier tube, a photodiode, a photodiode array or a charge-coupled device (CCD). Single photodiode detectors and photomultiplier tubes are used with scanning monochromators, which filter the light so that only light of a single wavelength reaches the detector at one time. The scanning monochromator moves the diffraction grating to "step-through" each wavelength so that its intensity may be measured as a function of wavelength. Fixed monochromators are used with CCDs and photodiode arrays. As both of these devices consist of many detectors grouped into one or two dimensional arrays, they are able to collect light of different wavelengths on different pixels or groups of pixels simultaneously. Common incandescent or quartz halogen light bulbs are most often used as broadband sources of near-infrared radiation for analytical applications. Light-emitting diodes (LEDs) are also used. The type of detector used depends primarily on the range of wavelengths to be measured.

The primary application of NIR spectroscopy to the human body uses the fact that the transmission and absorption of NIR light in human body tissues contains information about hemoglobin concentration changes. By employing several wavelengths and time resolved (frequency or time domain) method and/or spatially resolved methods, blood flow, volume and absolute tissue saturation ($StO_2$ or Tissue Saturation Index (TSI)) can be quantified. Applications of oximetry by NIRS methods include neuroscience, ergonomics, rehabilitation, brain computer interface, urology, the detection of illnesses that affect the blood circulation (e.g., peripheral vascular disease), the detection and assessment of breast tumors, and the optimization of training in sports medicine.

With respect to absorption spectroscopy, the Beer-Lambert law states that the absorbance of a solution is directly proportional to the concentration of the absorbing species in the solution and the path length. Thus, for a fixed path length, UV/Vis and NIR spectroscopy can be used to determine the concentration of the absorber in a solution. The method is most often used in a quantitative way to determine concentrations of an absorbing species in solution, using the Beer-Lambert law: $A = \log_{10}(I_0/I) = \varepsilon c L$

where A is the measured absorbance, in **A**bsorbance **U**nits (**AU**),
$I_0$ is the intensity of the incident light at a given wavelength,
I is the transmitted intensity,
L the path length through the sample, and
c the concentration of the absorbing species.

For each species and wavelength, $\varepsilon$ is a constant known as the molar absorptivity or extinction coefficient. This constant is a fundamental molecular property in a given solvent, at a particular temperature and pressure, and has units of 1/M*cm or often AU/M*cm. The absorbance and extinction $\varepsilon$ are sometimes defined in terms of the natural logarithm instead of the base-10 logarithm.

The Beer-Lambert Law is useful for characterizing many compounds but does not hold as a universal relationship for the concentration and absorption of all substances.

It is recognized by those skilled in the art that various factors affect these spectroscopic systems. These factors include spectral bandwidth, wavelength error, stray light, deviations from the Beer-Lambert law, and measurement uncertainty sources.

Stray light is an important factor that affects spectroscopic systems. Stray light causes an instrument to report an incorrectly low absorbance.

Deviations from the Beer-Lambert law arise based on concentrations. At sufficiently high concentrations, the absorption bands will saturate and show absorption flattening. The absorption peak appears to flatten because close to 100% of the light is already being absorbed. The concentration at which this occurs depends on the particular compound being measured.

Measurement uncertainty arises in quantitative chemical analysis where the results are additionally affected by uncertainty sources from the nature of the compounds and/or solutions that are measured. These include spectral interferences caused by absorption band overlap, fading of the color of the absorbing species (caused by decomposition or reaction) and possible composition mismatch between the sample and the calibration solution. From US 2015/0316471 A1 an apparatus for detecting a component in a sample is known which comprises two light sources emitting light at different wavelengths and arranged along an optical path in front of a sample chamber. The absorbance of the light emitted by the second light source in the sample chamber is used to estimate a path length through the sample chamber. The absorbance of the light emitted by the first light source in the sample chamber and the estimated path length are used to determine the hemoglobin concentration of the sample. The apparatus further comprises a xenon lamp which is used for calibrating the wavelength of the spectrometer.

## SUMMARY OF THE INVENTION

It is known that human hemoglobin (HGB) is an oxygen carrying protein in erythrocytes. The determination of its concentration in whole blood is a useful and important diagnostic tool in clinical biochemistry. COOx analyzers are used to measure the hemoglobin parameters of blood, such as total hemoglobin (tHb), carboxyhemoglobin (COHb), deoxyhemoglobin (HHb), oxyhemoglobin (O2Hb), methemoglobin (MetHb), and fetal hemoglobin (FHb) as well as total bilirubin (tBil) using optical absorbance measurements. In practice, typical COOx analyzers use lysed blood instead of whole blood because of the problems encountered with spectrometric analysis of whole blood. The measurement of lysed blood is relatively straightforward since the lysing process dissolves the red blood cells and turns the blood into an almost non-diffusing medium. The absorbance is measured with a simple collimated beam through the cuvette with little loss of light due to scattering. Because of the low loss of light due to scattering, a straightforward linear analysis may be used to find the hemoglobin and total bilirubin parameters.

Measurement of hemoglobin and total bilirubin parameters using a whole blood sample is very challenging due to the strong optical scattering of whole blood. These problems are primarily related to handling the increased light scattering level of whole blood as compared to lysed blood. This introduces light loss and nonlinear absorbance into the measurement.

The components in a prism-based spectrometer naturally have a low stray light profile. The major contributing factor to stray light performance is related to how the components are used.

Although the problems are primarily related to handling the increase light scattering level of whole blood, it is not a single factor that, if resolved, is capable of solving these difficult problems. The inventors have identified several factors that need to be addressed in order to measure hemoglobin parameters in whole blood. Because whole blood is a very diffuse medium, it is necessary to collect as much light as possible to reduce the requirement for an upper absorbance measurement range. It is also necessary to expand the upper limit of the measured absorbance due to the lower range of detector linearity correction. Blood settling effects are another problem that leads to poor correlation of absorbance of whole blood scans to absorbance of lysed blood scans. Basically, the blood cells are forming clumps or rouleaux. LED white light source brightness must also be increased. Lastly, new algorithms other than linear-based algorithms are needed to overcome the light scattering effects of whole blood.

Typical collection optics for systems using lysed blood are designed to collect light from the cuvette in a cone of about +/-0.7 degrees wide and have an upper measure absorbance limit of 1.5 A.U. (absorbance units). It was discovered by the inventors that for whole blood the system needs to collect light from the cuvette in a cone of about +/-12 degrees and that the upper absorbance limit had to increase to about 3.5 A.U. As for blood settling effects, the typical time it takes to measure the absorbance spectrum (approx. 1 minute), the whole blood in the cuvette is settling and the blood cells are forming clumps or rouleaux. Consequently, the scattering effects and the absorbance change with time.

The inventors discovered that changing the spectrometer control to collect multiple scans frequently rather than a few scans averaged over a longer period avoided step functions in the composite absorbance scan, which is stitched together from scans from several integration times. Unfortunately, adding more scans to expand the absorbance upper limit increases the data collection time. To resolve this dilemma, integration time was lowered from 5 msec to 1.2 msec to reduce data collection time. It was discovered, however, that this only works if the light level is increased by a corresponding factor. Thus, the LED white light brightness must be increased.

The optical absorbance measurement of a diffuse sample such as whole blood presents a unique problem. The diffuse transmittance of the whole blood sample scrambles the initial spatial light distribution of the measurement system caused by the non-uniformity typical of light sources. Thus, the spatial light distribution of the "blank" scan can be quite different from the whole blood sample scan. Since optical detectors have response that varies spatially, the response can vary due to spatial distribution changes of the incident light, even if the overall intensity has not changed. An absorbance scan which is based on the ratio of the whole blood sample scan to the blank scan will have a significant absorbance component due to this this non-uniformity of the light source in addition to the absorbance due to the sample alone. This results in a significant measurement error of the whole blood sample absorbance that is intolerable for cooximetry.

It was discovered that, by placing the sample cuvette between diffusers, the spatial light distribution appears the same for the blank and sample scans, thus, removing this error effect. The diffusers are specially chosen so that they diffuse a ray of incident light into the full acceptance cone of the optical system, but not more, so that as much light throughput as possible may be preserved while scrambling the ray completely across the field.

In addition, the measurement of fetal hemoglobin parameters presents additional problems. These include spectral acquisition times, which must be faster. Instead of the typical 12 seconds, it must be 5 seconds or less. The spectral acquisition time includes integration time multiplied by the number of coadded spectra and the processing time to produce one spectrum (full light, dark or sample) meeting all the following requirements. Absolute wavelength accuracy must be less; less than +0.03/-0.03 nm compared to +0.1/-0.0 nm. Wavelength calibration maintenance (less than +0.06/-0.0 nm versus +0.1/-0.0 nm), wavelength calibration drift (less than 0.024 nm/°C compared to 0.04 nm/°C), dark current level (less than 0.06%/°C for maximum dynamic range versus 0.1%/°C of maximum dynamic range), response nonlinearity (less than 0.06% after correction and less than 1.2% for lowest and highest 10% of dynamic range compared to 0.1% after correction and 2.0% for lowest and highest 10% of dynamic range), scattered light level (less than 0.02% of maximum dynamic range for fully illuminated detector array versus 0.1% of maximum dynamic range for fully illuminated detector array), thermal drift of response (intensity change maximum of 6% and tilt max of 6% over spectral range compared to intensity change maximum of 10% and tilt max of 10% over spectral range), and temperature excursion allowed during measurement (less than 0.5°C compared to 2°C) must all be less.

Commercially available compact and low-cost spectrometers typically use diffraction gratings (reflective or transmissive) to disperse the light input. Diffraction gratings give a high degree of dispersion in a small volume, and produce a relatively constant bandwidth (or resolution) vs. wavelength preferred by the typical user. Gratings, however, suffer from high stray light due to multiple diffraction orders and also from the imperfections inherent in the lines that are etched to produce the grating surface. Thus, mass-produced but expensive master holographic gratings are typically employed in applications requiring low stray light, rather than the more commonly available replicated gratings.

The requirement for low stray light for COOx analyzers limits the population of suitable grating manufacturers to the several in the world that produce master holographic or individually precision photoetched gratings. This serves to make it difficult to get low-cost high-performance gratings in quantity.

Prisms are also used to make spectrometers. Prisms have no issues with multiple diffraction orders and their surfaces have orders of magnitude fewer imperfections than the surface of a grating. The components in a prism-based spectrometer naturally have a low stray light profile. Thus, stray light in a prism spectrometer can potentially be lower by an order of magnitude or more compared to a grating spectrometer of otherwise similar design. The major contributing factor to stray light performance arises from how the components are used. There are three main sources of stray light. These include (1) overfilling of the spectrometer numerical aperture, (2) retroreflection from the light-array detector, and (3) the focal plane image. Light in excess of that required to fully illuminate the numerical aperture of the spectrometer can bounce around in the spectrometer and land on the detector. The numerical aperture of the optical fiber is 0.22 and the numerical aperture of the prism spectrometer is 0.1. A stop placed above the optical fiber input restricts the light input cone from the optical fiber to prevent excess light input. The light-array detector does not absorb all of the light impinging upon it, but back-reflects a portion. This retroreflection must be controlled to land into an absorbing surface or beam trap to prevent it from scattering onto the detector. Imparting a slight tilt of the light-array detector forces the retroreflection back into a harmless direction. The image of the slit on the detector focal plane must be as sharp as possible. Any excessive overfill of the detector due to defocus can be a potential source of stray light. If this light hits detector structures such as bond wires, metallization pads, etc., it can bounce back onto the sensitive surface of the detector.

Additionally, a prism spectrometer spreads the blue end of the spectrum out over more pixels than a diffraction grating spectrometer and, thus, the blue end of the spectrum gives a lower signal per pixel. To compensate for the lower signal per pixel, an LED with higher blue power, or a cool-white LED, is used. The signal in the blue can be further boosted by adding an inexpensive filter glass after the LED that slightly attenuates the red end. Kopp filter glass type 4309, about 3 mm thick, is useful for this purpose. The main disadvantage of prisms is the lower dispersive power they have compared to a grating, and the variation of resolution with wavelength. In embodiments of the present invention when a prism is used, the former disadvantage is mitigated by using a small enough light-array detector; the latter is mitigated because the analysis of whole blood does not require a uniformly small resolution across the waveband of interest.

Currently available spectrometers typically list a uniform 1 nm resolution for the blood measurement spectral region of 455-660 nm. In embodiments of the present invention, the spectral region is expanded and covers the spectral region of 422-695 mm. Further, the resolution is selectively changed upward in regions where low resolution is not required (such as the 600-695 nm region and 422-455 nm region). In embodiments of the present invention, these regions have a resolution greater than 1 nm. Typically, the resolution is about 3.0 to about 3.5 nm. These ranges are used to capture additional wavelength calibration peaks for wavelength calibration and fluid detection. The larger spectral region of embodiments of the present invention requires consideration of the dispersed spectrum from the prism. The dispersed spectrum must be spread out over the light-array detector and cover enough pixels to sample the spectrum at a fine enough resolution but not so much as to extend outside of the detector array. Due to the wider spectral range, embodiments of the present invention incorporate a light-array detector having 1024 pixels with an active area length of about 8.0 mm.

A minimal-part reference design for an optical dispersion spectrometer requires only two optical components: a light dispersion element (i.e. prism or grating) and a doublet (achromatic) lens. The prism/grating has a reflective coating on the base. One example of an acceptable prism is a Littrow prism. The Littrow prism has a structure such that it is usable for a compact and low-cost spectrometer of embodiments of the present invention. The prism material (dispersion characteristic) and the lens focal length are further considerations. Although other prisms and achromatic lenses may be used, one embodiment of the present invention incorporates a Schott F5 glass prism and an 80 mm focal length lens. This particular combination provides a dispersion length of the spectrum of about 6.48 mm. This dispersion length leaves about 0.75 mm on either end of the light-array detector available for tolerance variations and dark correction pixels.

Thermal drift of the spectral response must be considered. It is critical that the spectral response of the spectrometer stays within a certain range between the full light and whole blood scans. Any change in spectrometer response will cause absorbance errors. The main precaution against this change is to make sure that the image of the slit overfills the pixels so that image drift due to temperature does not cause a reduction of light on the detector pixel. The 1:1 imaging of the system combined with a 200 $\mu$m diameter optical fiber overfills the 125 $\mu$m tall pixels. As long as image drift is confined to less than about 30 $\mu$m of movement in either direction along the detector over a measurement interval, thermal drift is not a problem. The present invention also contemplates various mechanisms to minimize thermal drift effects on the spectral response. These mechanisms include insulating the spectrometer housing to minimize temperature changes external to the spectrometer housing, maintaining the temperature within the spectrometer housing using a temperature-controlled heat source, and/or incorporating a temperature-compensating lens mount for the achromatic lens.

The process of the present invention that transforms the electrical signals from the spectrometer will now be discussed. First, the absorbance is measured, which is minus the base-ten logarithm of the ratio of the electrical signal received when the blood sample is in the cuvette to the electrical signal received when a clear fluid is in the cuvette. Second, the absorbance values at each wavelength are put into a mapping function that maps absorbance values to the analyte levels (COOx parameters and bilirubin) in the whole blood sample. The mapping function and its coefficients are established by using the absorbance values measured for whole blood samples with known analyte values, and establishing the relationship between these absorbance values and the known analyte values.

Embodiments of the present invention achieve these and other objectives by providing a compact, low-cost COOx analyzer subsystem.

In one embodiment of the present invention, there is an optical absorbance measurement system for measuring whole-blood hemoglobin parameters with the features of claim 1. The system includes, inter alia, (a) an optical-sample module having a light-emitting module, a replaceable cuvette assembly, and a calibrating-light module, (b) an optical fiber, (c) a spectrometer module, and (d) a processor module. The light-emitting module has an LED light source capable of emitting light where the light is directed along an optical path. The cuvette assembly is adjacent the light-emitting module where the cuvette assembly is adapted for receiving a whole-blood sample and has a sample receiving chamber with a first cuvette window and a second cuvette window aligned with each other. The sample receiving chamber is disposed in the optical path for receiving light from the LED light source and has a defined optical path length between the first cuvette window and the second cuvette window along with an electronic chip capable of storing a path-length value of the sample receiving chamber. The calibrating-light module has a calibrating-light source with one or more known wavelengths of light where the calibrating-light module is capable of emitting a calibrating light into the optical path. The optical fiber has a light-receiving end and a light-emitting end. The light-receiving end optically connects to the optical-sample module where the light-receiving end receives the light from the optical path and conducts the light to the light-emitting end. The spectrometer module receives the light from the light-emitting end of the optical fiber, separates the light into a plurality of light beams where each light beam has a different wavelength, and converts the plurality of light beams into an electrical signal. The processor module (1) obtains the path-length value of the sample receiving chamber of the replaceable cuvette from the electronic chip and (2) receives and processes the electrical signal from the spectrometer module generated for a whole-blood sample. The path-length value of the sample chamber is used to transform the electrical signal into an output signal useable for displaying and reporting hemoglobin parameter values and/or total bilirubin parameter values for the whole-blood sample.

In another embodiment of the present invention, the light-emitting module includes a plurality of optical components disposed in the optical path between the LED light source and the cuvette assembly where the plurality of optical components includes at least the first optical diffuser and one or more of a collimating lens, a circular polarizer, and a focusing lens.

In the embodiment of the present invention, the calibrating-light module includes a second diffuser disposed in the optical path downstream from the cuvette assembly but upstream from a beam splitter.

In another example , there is disclosed an optical absorbance measurement system for whole blood. The system includes an optical-sample module, an optical fiber, a spectrometer module, and a processor module. The optical-sample module includes a light-emitting module, a cuvette module, a first optical diffuser, and a second optical diffuser. The cuvette module is positioned between the first optical diffuser and the second optical diffuser. The spectrometer module receives the light from the light-emitting end of the optical fiber, separating the light into a plurality of light beams and converting the plurality of light beams into an electrical signal. The processor module receives and processes the electrical signal from the spectrometer module generated for the whole-blood sample and transforms the electrical signal into an output signal useable for displaying and reporting hemoglobin parameter values and/or total bilirubin parameter values for the whole-blood sample.

In yet another embodiment, the spectrometer module includes an input slit positioned in the optical path to receive the light emitted from the light-emitting end of the optical fiber and to transmit the light therethrough, a light dispersing element disposed in the optical path where the light dispersing element receives the light transmitted through the input slit, separates the light into the plurality of light beams where each light beam has a different wavelength, and re-directs the plurality of light beams back toward but offset from the input slit, and a light-array detector capable of receiving the plurality of light beams and converting the plurality of light beams into an electrical signal for further processing.

In another example, the spectrometer module has a thermal-compensating means for maintaining a position of the plurality of light beams on the light-array detector. The thermal-compensating means includes one or more of insulation disposed around the spectrometer housing, a temperature controller assembly disposed on the spectrometer housing (the temperature controller assembly being, for example, a heating tape with a thermistor or other temperature measuring component and a program that controls the heating of the tape based on the temperature within the spectrometer housing), and a thermal-compensating lens mount.

In a still further example, the thermal-compensating lens mount has a fixed mount end and an unfixed mount end that permits thermal expansion and contraction of the thermal-compensating lens mount. The fixed mount end is fixedly attached to a baseplate or a bottom of the spectrometer housing. The lens mount has a coefficient of expansion greater than the coefficient of expansion of the baseplate or the spectrometer housing to which the lens mount is attached. The thermal-compensating lens mount moves linearly and transversely relative to an optical path of the light from the light input slit based on the coefficient of expansion of the lens mount. This temperature-based movement of the lens mount maintains the position of the dispersed light from the light dispersing element onto the light-array detector. In other words, thermal re-positioning of the achromatic lens by way of the thermal-compensating lens mount causes the dispersed light from the light dispersing element to impinge onto the light-array detector without affecting the electric signal generated by the light-array detector from the impinging light. The shift of the light beam is caused by the light-dispersing element reacting to a temperature change.

In another example, there is disclosed a compact spectrometer for measuring hemoglobin parameters in whole blood. The spectrometer includes an enclosed housing having a light input end/an optical fiber housing end with a light entrance port, a light input slit disposed on an electronic circuit substrate, the electronic circuit substrate disposed in the enclosed housing where the light input slit is aligned with and adjacent to the light entrance port, a light-array detector disposed on the circuit board substrate adjacent the light input slit, and an optical component group consisting of a light dispersing element disposed downstream from the light input slit and a spherical achromatic lens disposed between the light input slit and the light dispersing element where the light dispersing element has a reflective surface on a back side to reflect the dispersed light back toward the achromatic lens. The achromatic lens transmits light from the light input slit to the light dispersing element and transmits dispersed light reflected from the light dispersing element to the light-array detector. To accomplish this, the achromatic lens is slightly off axis relative to the light coming from the light input slit so that the dispersed light from the light dispersing element is not directed back to the light input slit but to the light-array detector.

In a further embodiment, there is disclosed a method of measuring whole-blood hemoglobin parameters despite strong optical scattering caused by whole blood. The method includes providing a light source such as a LED light source with a spectral range of about 422 nm to about 695 nm, guiding light having the spectral range from the light source along an optical path, providing a cuvette module with a sample receiving chamber having a first cuvette window disposed in the optical path where the first cuvette window transmits the light through the sample receiving chamber and through a second cuvette window aligned with the first cuvette window where the sample receiving chamber contains a sample of whole blood, providing a pair of diffusers (i.e. a first diffuser and a second diffuser) disposed in the optical path where the first cuvette window and the second cuvette window of the sample receiving chamber of the cuvette are disposed between the pair of diffusers, guiding light from the cuvette module into a spectrometer having a light dispersing element that separates the light into a plurality of light beams where each light beam has a different wavelength and converts the plurality of light beams into an electrical signal, and processing the electrical signal into an output signal useable for displaying and reporting hemoglobin parameter values and/or total bilirubin parameter values of the sample of whole blood.

In another embodiment of the method, the processing step includes processing the electrical signal to spectral absorbance and then mapping the spectral absorbance to hemoglobin parameter values and/or bilirubin parameter values using a computational mapping function.

In still another embodiment of the method, the processing step includes using a kernel-based orthogonal projection to latent structures mapping function as the computational mapping function.

In another example, there is disclosed a method of measuring hemoglobin parameters in a whole blood sample. The method includes (1) measuring and recording a transmitted light intensity scan over a plurality of wavelengths in a measurement range by transmitting light through a cuvette module having an optical path with a known optical path length therethrough where the cuvette module is filled with a transparent fluid, (2) measuring and recording a transmitted light intensity scan over the plurality of wavelengths of the measurement range by transmitting light through the cuvette a second time having the optical path with the known optical path length therethrough where the cuvette module is filled with a whole blood sample, wherein each measuring and recording step of the transparent fluid and the whole blood sample includes diffusing and circularly polarizing the transmitted light before transmitting the transmitted light through the cuvette module and then diffusing the transmitted light emitting from the cuvette module before determining a spectral absorbance, (3) determining a spectral absorbance at each wavelength of the plurality of wavelengths of the measurement range based on a ratio of the transmitted light intensity scan of the whole blood sample to the transmitted light intensity scan of the transparent fluid using a prism-based spectrometer, and (4) correlating the absorbance at each wavelength of the plurality of wavelengths of the measurement range to hemoglobin parameter values and/or bilirubin parameter values of the blood sample using a computational mapping function.

## BRIEF DESCRIPTION OF THE DRAWINGS

FIGURE 1 is a simplified, perspective view of one embodiment of the present invention showing a compact COOx subsystem.

FIGURE 2 is a side elevation view of one embodiment of an optical-sample module shown in Fig. 1.

FIGURE 3 is a front, perspective view of one embodiment of a light-emitting module of the optical-sample module shown in Fig. 2.

FIGURE 3A is a front, perspective view of the light-emitting module shown in Fig. 3 showing a plurality of optical components.

FIGURE 3B is an enlarged, side elevation view of the optical components shown in Fig. 3A.

FIGURE 4 is a front perspective view of one embodiment of a cuvette assembly of the optical-sample module shown in Fig. 1.

FIGURE 5 is a rear perspective view of the cuvette assembly shown in Fig. 4.

FIGURE 6 is a front elevation view of a cuvette module of the cuvette assembly showing fluid input and output ports, a sample receiving chamber, a sample window, and an electronic chip assembly.

FIGURE 7 is a rear perspective view of the sample receiving chamber of Fig. 6 showing cuvette first and second windows.

FIGURE 8 is a rear plan view of the sample receiving chamber showing the electronic chip assembly disposed adjacent the sample receiving chamber.

FIGURE 9 is a perspective view of one embodiment of a calibrating light module of the optical-sample module of Fig. 1.

FIGURE 10 is a side cross-sectional view of the calibrating light module of Fig. 8 showing a calibrating light source.

FIGURE 11 is a simplified, side plan view of the calibrating light source of the calibrating light module of Fig. 9 showing a plurality of optical components.

FIGURE 12 is a front perspective view of one embodiment of a spectrometer module of Fig. 1 with a cover removed showing the internal components.

FIGURE 13 is a rear perspective view of the spectrometer module of Fig. 12 showing an input light slit and adjacent light-array detector.

FIGURE 14 is a rear cross-sectional view of the spectrometer module of Fig. 12 showing a single circuit board and the location of the input light slit and the light-array detector.

FIGURE 15 is a top view of the spectrometer module of Fig. 12 showing the optical components with superimposed ray trace.

FIGURE 16 is a ray trace showing the input light from the input light slit and a plurality of light beams refracted onto the light-array detector.

FIGURE 17A is a perspective view of one embodiment of a thermal-compensating means for the spectrometer module showing insulation wrapped around the spectrometer module.

FIGURE 17B is a perspective view of another embodiment of a thermal-compensating means for the spectrometer module showing a temperature controlling assembly.

FIGURE 17C is a cross-sectional view of one embodiment of a lens mount of the spectrometer module of Fig. 12 showing a termperaturecompensating lens mount.

FIGURE 18 is a cross-sectional view of one embodiment of a lens mount of the spectrometer module of Fig. 12 showing a fixed lens mount.

FIGURE 19 is a graphic illustration showing the correlation results of the COOx analyzer subsystem for total hemoglobin using a KOPLS mapping function and method.

FIGURE 20 is a graphic illustration showing the correlation results of the COOx analyzer subsystem for oxyhemoglobin using a KOPLS mapping function and method.

FIGURE 21 is a graphic illustration showing the correlation results of the COOx analyzer subsystem for carboxyhemoglobin using a K-OPLS mapping function and method.

FIGURE 22 is a graphic illustration showing the correlation results of the COOx analyzer subsystem for deoxyhemoglobin using a K-OPLS mapping function and method.

FIGURE 23 is a graphic illustration showing the correlation results of the COOx analyzer subsystem for methemoglobin using a KOPLS mapping function and method.

FIGURE 24 is a graphic illustration showing the correlation results of the COOx analyzer subsystem for total bilirubin using a KOPLS mapping function and method.

## DETAILED DESCRIPTION

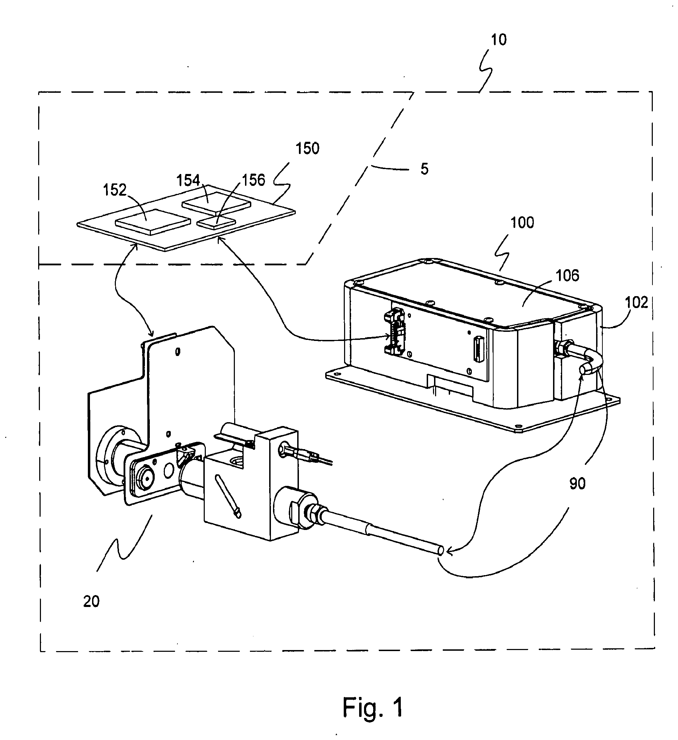

Embodiments of the present invention are illustrated in Figs. 1-24. Figure 1 shows one embodiment of a COOx analyzer subsystem 10. COOx analyzer subsystem 10 includes at least an optical-sample module 20, an optical fiber 90 and a spectrometer module 100. COOx analyzer subsystem 10 may optionally include a processor module 150 or processor module 150 may optionally be included in an electronics circuit of a diagnostic system in which the COOx analyzer subsystem 10 is a part. Line 5 is included to signify that the processor module 150 may or may not be part of the COOx subsystem 10. Processor module 150 includes, but is not limited to a microprocessor module152 and a memory module 154. Optionally, the processor module 150 may also include a converter module 156 or converter module 156 may be external to the COOx analyzer subsystem 10. COOx analyzer subsystem 10 is used to measure the hemoglobin parameters of blood such as total hemoglobin (tHb), carboxyhemoglobin (COHb), deoxyhemoglobin (HHb), oxyhemoglobin (O2Hb), methemoglobin (MetHb), and fetal hemoglobin (FHb) as well as total bilirubin (tBil) using optical absorbance.

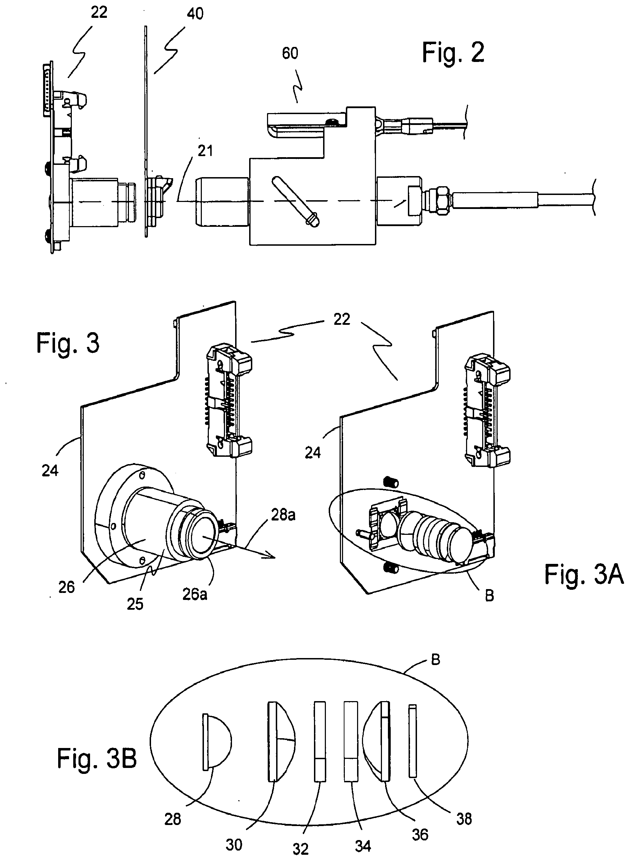

Figure 2 illustrates optical-sample module 20. Optical-sample module 20 includes a light-emitting module 22, a cuvette assembly 40 and a calibrating-light module 60. Light-emitting module 22, as the term implies, emits a visible light beam toward the cuvette assembly 40 that is then received by the calibrating-light module 60, which is then transmitted to spectrometer module 100. The light beam 12 defines an optical path 21.

Figures 3-3A illustrate perspective views of the embodiment of light-emitting module 22 of Fig. 2. Light-emitting module 22 includes a light-emitting module substrate 24 that contains an electrical circuit (not shown) and a light-emitting optics assembly 25. Light-emitting optics assembly 25 has an optics assembly housing 26 with an optics assembly end 26a. A beam of visible light 28a emits from optics assembly end 26a of light-emitting optics assembly 25 when light-emitting module 22 is powered on by a signal received from processor module 150. Fig. 3A illustrates light-emitting optics assembly 25 with optics assembly housing 26 removed exposing a plurality of optical components B contained within light-emitting assembly 25.

Turning now to Figure 3B, there is illustrated an enlarged side view of the plurality of optical components B of Fig. 3A. In this embodiment, optical components B includes a light-emitting diode (LED) light source 28, a collimating lens 30, a first diffuser 32, a circular polarizer 34, a focusing lens 36, and an optional protective window 38. Circular polarizer 34 provides a distinct advantage. This advantage provides improved sensitivity and accuracy of the system. Hemoglobin has optical rotary characteristics, which means that the polarization sensitivity of a spectrometer will cause an absorbance error if noncircularly polarized light is used to measure hemoglobin absorbance. Unlike for other polarization states of light, the polarization state of the circularly polarized light is not changed when passing through hemoglobin. Thus, the polarization response of the spectrometer is the same for the circularly polarized light passing through the hemoglobin as it is for the reference scan taken with the cuvette filled with a transparent fluid.

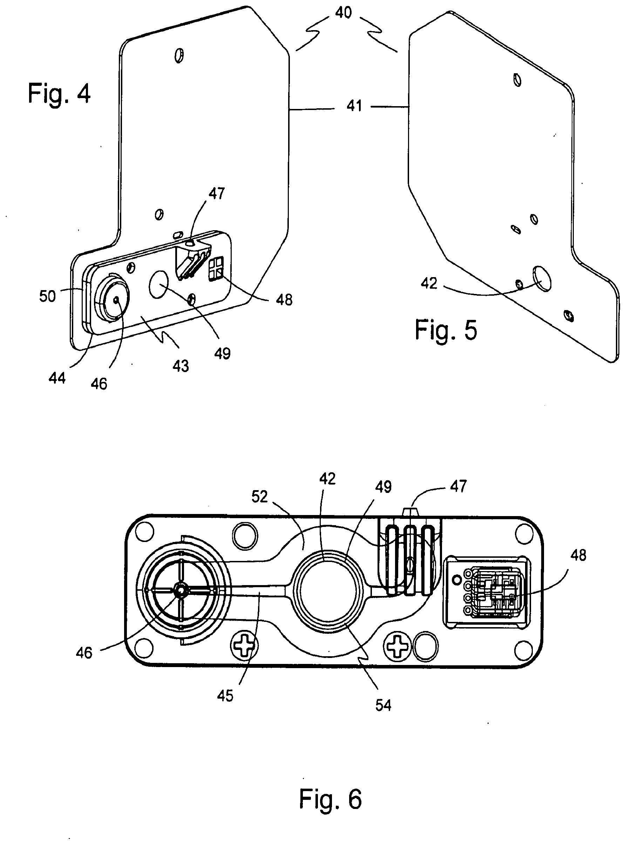

Figures 4 and 5 illustrated front and rear perspective views of one embodiment of the cuvette assembly 40. Cuvette assembly 40 includes a cuvette substrate 41 and a cuvette module 43. Cuvette substrate 41 provides a support for securing the cuvette assembly 40 within the analyte subsystem 10 and includes a cuvette light path opening 42 that is disposed within optical path 21 and is aligned with the light beam emitted from light-emitting module 22. Cuvette module 43 includes a cuvette first portion 44 having a sample receiving recess 45, a sample inlet port 46, a sample outlet port 47, an electronic chip assembly 48, and a first cuvette window 49, and a cuvette second portion 50 having a second cuvette window 52 (shown in Fig. 6 and delineated as outline 53) opposite and aligned with the first cuvette window 49 where the first and second cuvette windows 49, 52 are aligned with and dispersed within optical path 21. Cuvette first portion 44 and cuvette second portion 50 are bonded to each with or without a gasket disposed between cuvette first and second portions 44, 50. Bonding may be achieved using adhesives, ultrasonic techniques, solvent based techniques, etc. When assembled and as shown in Fig. 6, sample receiving recess 45 of cuvette first portion 44 forms a sample receiving chamber 54 with cuvette second portion 50 that fluidly communicates with sample inlet and outlet ports 46, 47. The distance between first and second cuvette windows 49, 52 of sample receiving chamber 54 define a cuvette optical path length, which is accurately measured and stored within electronic chip 48 for later retrieval by processor module 150. A typical optical path length used in this embodiment of the present invention is 0.0035 inches (0.090 mm).

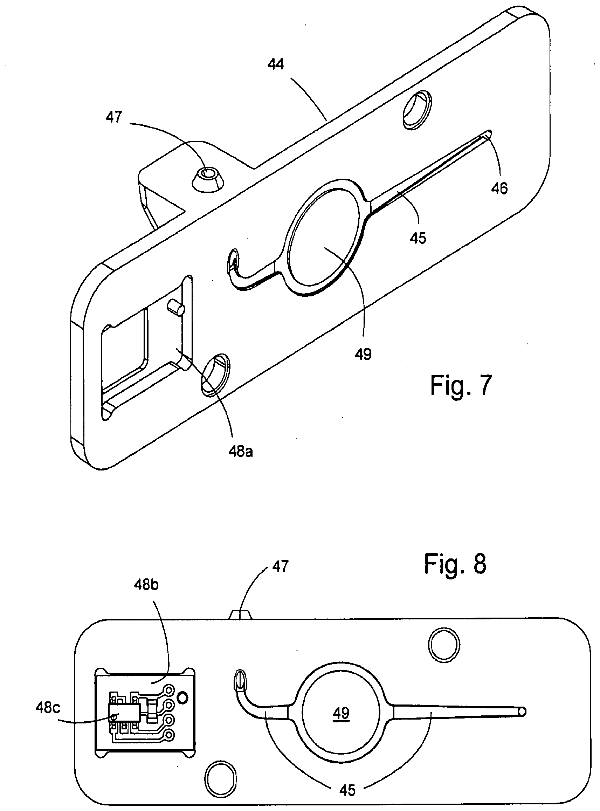

Turning now to Figure 7, there is illustrates an enlarged, rear perspective view of cuvette first and second portions 44, 50. As shown, cuvette first portion 44 has sample chamber recess 45 with first cuvette window 49 and electronic chip recess 48a for receiving electronic chip assembly 48. Cuvette second portion 50 has second cuvette window 52 that forms sample receiving chamber 54 when assembled together with cuvette first portion 44. Second cuvette window 52 as delineated by an outline 53 on cuvette second portion 50 is a raised surface that forms a water-tight seal around sample chamber recess 45 and sample receiving chamber 54. Optionally, a thin gasket may be positioned between cuvette first and second portions 44, 50 to more easily ensure a water-tight seal. Figure 8 shows a rear view of cuvette first portion 44 with electronic chip assembly 48 disposed within electronic chip recess 48a. Electronic chip assembly 48 includes a chip circuit board 48b and an electronic chip 48c that stores the cuvette optical path length value for the particular cuvette module 43. First cuvette window 49 is disposed within the optical path 21 and transmits the light beam passing through the sample to the calibrating light module 60, which then passes the light beam to the spectrometer module 100.

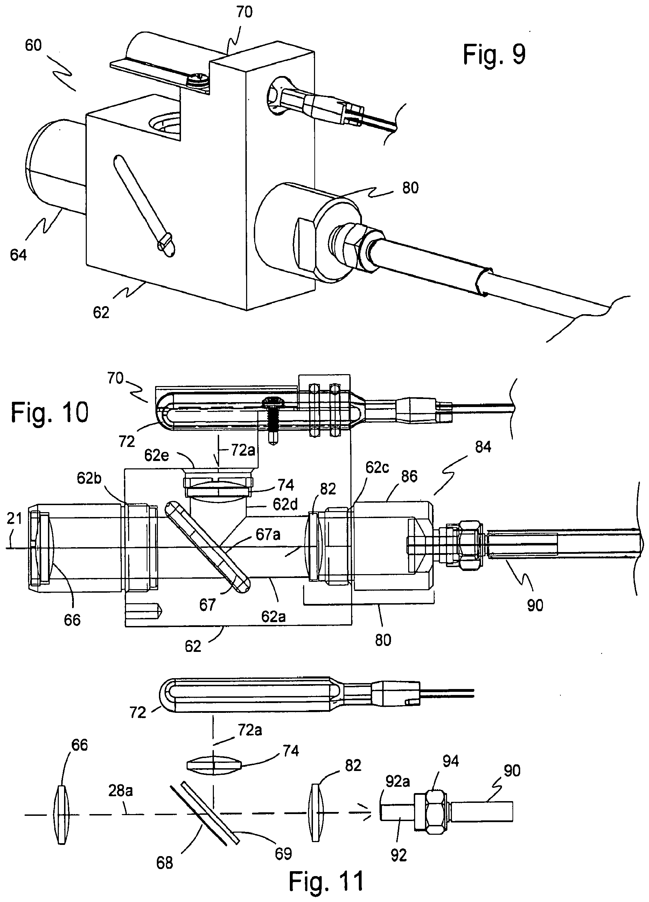

Turning now to Figure 9, there is illustrated one embodiment of the calibrating light module 60. Calibrating light module 60 includes a calibrating module housing 62, a light beam receiving portion 64, a calibrating light portion 70, and an optic fiber portion 80 where calibrating module housing 62, light beam receiving portion 64 and optic fiber portion 80 are aligned with optical path 21. Calibrating light portion 70 is spaced from and transverse to optical path 21.

Figure 10 is a cross-sectional, elevation view of calibrating light module 60. Calibrating module housing 62 includes a first tubular conduit 62a between a light beam input opening 62b and a light beam exit opening 62c as well as a second tubular conduit 62d that is transverse to and intersects with first tubular conduit 62a on one end and has a calibrating light beam opening 62e on an opposite end.

Light beam receiving portion 64 houses a collimating lens 66 that collimates light beam 28a received along optical path 21 from cuvette module 43 and directs light beam 28a into first tubular conduit 62a. Disposed within calibrating module housing 62 is beam splitter holder assembly 67 that is disposed transversely across first tubular conduit 62a. Beam splitter holder assembly 67 has an upward slanting surface 67a facing calibrating light beam opening 62e and light beam exit opening 62c within optical path 21. Beam splitter holder assembly 67 supports a second diffuser 68 and a beam splitter 69 (shown in Fig. 11) that is disposed downstream along optical path 21 from second diffuser 68 so that it is positioned to receive calibrating light beam 72a and direct it along optical path 21 and first tubular conduit 62a to light beam exit opening 62c.

Calibrating light portion 70 includes a calibrating light source 72 disposed adjacent but spaced from optical path 21 that is capable of directing a calibrating light beam 72a into calibrating module housing 62 through a calibrating light opening 62e transversely to optical path 21 toward beam splitter holder assembly 67. Within calibrating light portion 70, there is a collimating lens 74 that collimates calibrating light beam 72a before it is reflected by beam splitter assembly 67 toward light beam exit opening 62c.

Optic fiber portion 80 is located within optical path 21 at or in the vicinity of light beam exit opening 62c. Optic fiber portion 80 includes a focusing lens 82 and a optic fiber connector assembly 84 that includes a connector housing 86 adapted for receiving an optical fiber assembly 90. Optic fiber portion 80 is adapted to insure that light beam 28a is properly focused by focusing lens 82 into optical fiber assembly 90.

Figure 11 is a simplified illustration of Fig. 10 showing the positional relationship of the optical components 66, 68, 69, 74, 82 and light beams 28a, 72a as well as optical fiber assembly 90. As can be seen from Fig. 11, light beam 28a is received by collimating lens 66, transmitted through second diffuser 68 and beam splitter 69 to focusing lens 82 and into optical fiber assembly 90. As previously discussed, the importance of using a pair of diffusers (first diffuser 32 and second diffuser 68) with cuvette module 43 in between the pair of diffusers 32, 68 is that the spatial light distribution will appear the same for the blank scan and the whole blood sample scan. The use of diffusers 32, 68 in this arrangement removes the error effect caused by nonuniformity of the light source and/or variation in the spatial distribution changes of the incident light even if the overall intensity has not changed. Diffusers 32, 68 are chosen so that they diffuse a ray of incident light into the full acceptance cone of the optical component group 120 of the spectrometer module 100. This effectively scrambles the ray completely across the optical measuring field.

Calibrating light beam 72a when activated is received by collimating lens 74, transmitted to beam splitter 69 and directed to focusing lens 82 where it is focused into optical fiber assembly 90. Calibrating light beam 72a has specific wavelengths of light used for calibrating the wavelength scale of spectrometer module 100. One example of an acceptable calibrating light source 72 is a krypton (Kr) gas discharge lamp, which provides seven Kr line wavelengths in nanometers covering the range of 422 to 695 nm. Prism 131 of light dispersion component 130 has a nonlinear dispersion versus wavelength that requires a polynomial or other function of a higher order. An embodiment of the present invention uses a $5^{th}$ order polynomial to the pixel locations of the Kr line peaks to provide residual errors well below the absolute wavelength accuracy requirement of +/- 0.03 nm.

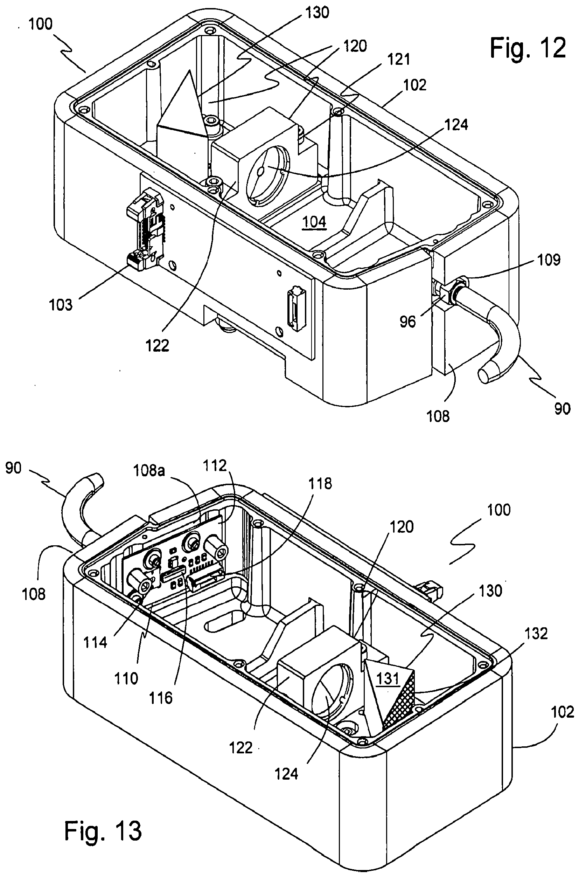

Optical fiber assembly 90 includes an optical fiber 92, a first optical fiber connector 94 and a second optical fiber connector 96 (shown in Fig. 12). First optical fiber connector 94 is secured to a light receiving end 92a of optical fiber 92 and directly and removably connects to connector housing 86 of optic fiber connector assembly 84. One embodiment of optical fiber 92 includes a 200 $\mu$m silica core fiber with a numerical aperture (NA) of 0.22.

Turning now to Figures 12 and 13, there is illustrated one embodiment of spectrometer module 100. Spectrometer module 100 includes a spectrometer housing 102, a spectrometer base 104, a spectrometer cover 106 (shown in Fig. 1), an optical fiber housing end 108, and an electrical signal output coupler 103. Spectrometer module 100 has an outside envelope dimension of 11 cm x 8 cm x 2 cm and optionally includes thermal compensation structures discussed later. Within spectrometer housing 102 are contained the essential components of spectrometer module 100. These components include a light-receiving and converting assembly 110 and an optical component group 120. Optical component group 120 includes an achromatic lens assembly 121 and a light dispersing element 130. Light dispersing element 130 may be a prism 131 or a grating 136. Optical fiber assembly 90 is removably secured to optical fiber housing end 108 at light entrance port 109, which optical fiber assembly 90 transmits the light beams 28a, 72a to spectrometer module 100. As previously mentioned, light beam 28a represents the light transmitted from light-emitting module 22 through cuvette module 43 whereas light beam 72a is the calibrating light transmitted from calibrating light module 60, which is used to calibrate spectrometer module 100.

Achromatic lens assembly 121 includes a lens mount 122 and a spherical achromatic lens 124. Achromatic lens 124 receives light beams 28a, 72a, as the case may be, and directs the light beam to light dispersion element 130, which in this embodiment is prism 131. Prism 131 has a reflective coating 132 on an outside back surface. Prism 130 refracts light beam 28a and reflects the light back through achromatic lens 124.

Light-receiving and converting assembly 110 is securely mounted adjacent an inside surface 108a of optical fiber housing end 108. Light-receiving and converting assembly 110 includes a circuit board substrate 112 upon which is mounted a light input slit 114 that is aligned with light-emitting end 92b (not shown) of optical fiber 92. Adjacent input slit 114 is a light-array detector 116 that receives the refracted light from prism 131. Light-array detector 116 converts the refracted light to an electrical signal, which is output through output connector 118 to processor module 150. Providing light input slit 114 and light-array detector 116 adjacent each other on circuit board 112 has several advantages. This feature greatly simplifies the construction and improves the precision of spectrometer module 100. Other spectrometers place these items on separate planes, where they have separate mounting structures, and have to be adjusted independently. This feature of mounting the input slit and light-array detector adjacent each other on circuit board 112 eliminates the need to mount and position each structure (i.e. slit and detector) separately.

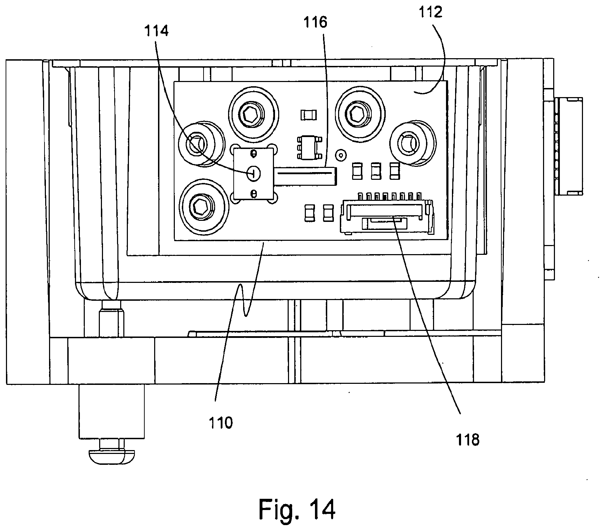

Figure 14 is an enlarged view of light-receiving and converting assembly 110. Light input slit 114 is 15 $\mu$m wide by 1000 $\mu$m long that projects an optical fiber-slit image that is a rectangle approximately 15 $\mu$m wide by 200 $\mu$m high onto the light-array detector 116 (Hamamatsu S10226-10 is an example of a usable light array detector). Input slit 114 is applied directly onto the same circuit board substrate 112 as and in close proximity to light-array detector 116. Light-array detector 116 has a pixel height between about 100 to about 150 $\mu$m, which allows a one-to-one imaging of the 200 $\mu$m diameter optical fiber onto the detector. In this embodiment, input slit 114 is laser etched in a precise position relative to light-array detector 116 making alignment less labor intensive. Because input slit 114 and light-array detector 116 are only slightly off-axis relative to the center axis of the achromatic lens 124, there is minimal aberration and a one-to-one imaging on light-array detector 116 is possible so that no cylindrical focusing lens is required to shrink the optical fiber image (200 μm diameter fiber) to match the pixel height of light-array detector 116.

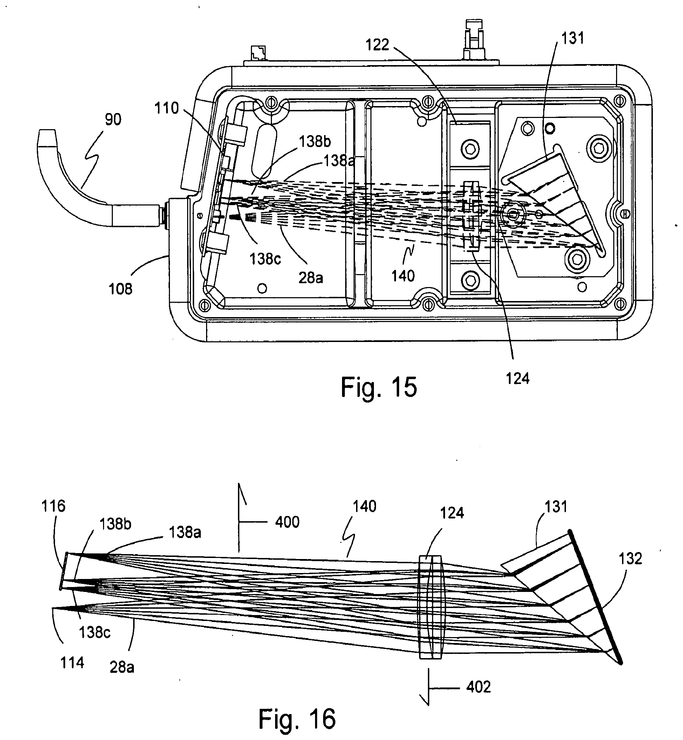

Turning now to Figure 15, there is a top view of spectrometer module 100 of Fig. 13. Superimposed onto Fig. 15 is a ray trace diagram 140 of the light beam delivered to spectrometer module 100 by optical fiber 92. As shown, light beam 28a enters spectrometer module 100 through input slit 114 toward achromatic lens 124. Achromatic lens 124 is used off-axis; that is, the achromatic lens is slightly off-axis to the light beam 28a. Light beam 28a is transmitted by achromatic lens 124 to prism 131, where light beam 28a is refracted into a plurality of light beams 138a, 138b, 138c of different wavelengths as prisms are ought to do. The plurality of light beams 138a, 138b, 138c are reflected by prism 131 back through achromatic lens 124. Achromatic lens 124 is used off-axis in order to direct the plurality of refracted and reflected light beams 138a, 138b, 138c from prism 131 onto light-array detector 116.

Figure 16 is an enlarged view of ray trace diagram 140. Achromatic lens 124 is used off-axis relative to entering light beam 28a. By using achromatic lens 124 off-axis along with prism 131 having a reflective coating 132 on a base of prism 131, there is achieved a compact, simplified, minimal-component spectrometer module 100 capable of being used for measuring hemoglobin parameters and/or total bilirubin parameters in whole blood.

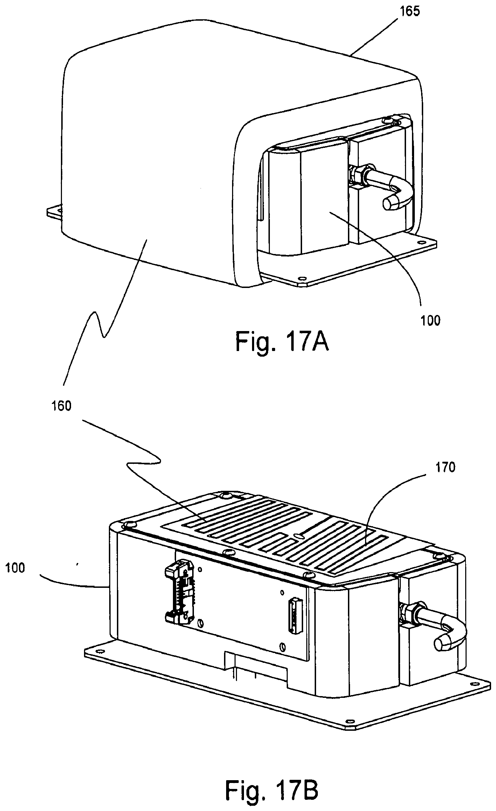

A change in temperature has a greater effect on beam refraction angle when using a prism instead of a diffraction grating. In an embodiment of the present invention, a thermal-compensating means 160 is provided to compensate for a thermal shift in the incoming light beam caused by the light-dispersing element 130. A temperature change within spectrometer module 100 causes a thermally-induced movement of the slit image from input slit 114 on light-array detector 116 caused in turn by thermally-induced changes in refractive index of the dispersive prism 131. Fig. 16 shows the direction of movement of the image on light-array detector 116 for the thermal refractive index change in prism 131 with arrow 400. If the lens 124 is moved in the opposite direction over the same temperature interval as indicated by arrow 402, the slit image will be moved back to where it should be onto light-array detector 116. To prevent this shift, the thermal-compensating means 160 may be a simple as wrapping spectrometer module 100 with insulation to minimize temperature change within spectrometer module 100 from a temperature change occurring outside of spectrometer module 100 or to place spectrometer module 100 within a temperature controlled space. Another means is to include a temperature controller assembly 170 that includes at least a ribbon heater 172 attached to an inside surface or an outside surface of the spectrometer housing 102 and a temperature sensor 174 such as thermocouple or thermistor to measure the temperature of the spectrometer housing and a heater circuit to maintain a predefined constant temperature. Figure 17A and 17B illustrate these possibilities.

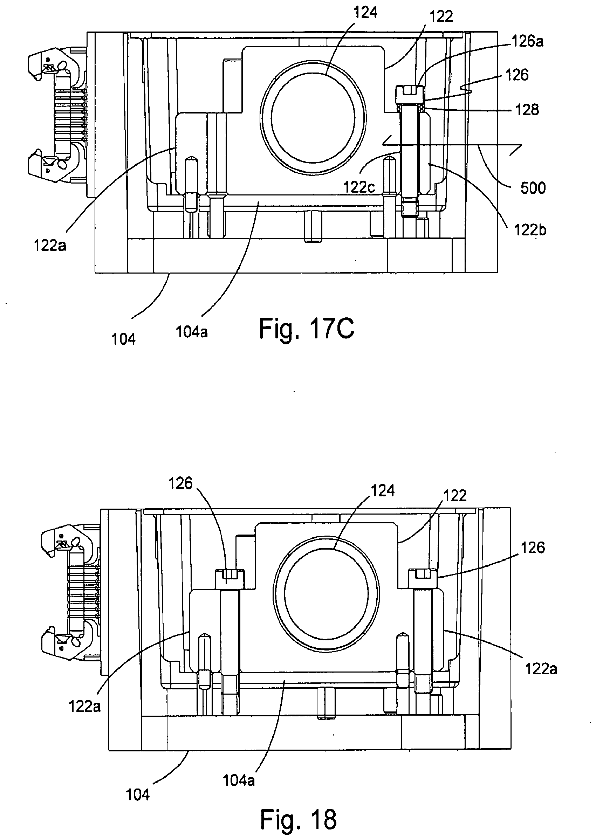

In one embodiment shown in Fig. 17C, achromatic lens mount 122 is a thermal-compensating lens mount. Thermal-compensating lens mount 122 has a fixed mount end 122a and an unfixed mount end 122b. Fixed mount end 122a is fixedly secured to spectrometer base 104 or a baseplate 104a that is securely attached to spectrometer base 104. Unfixed mount end 122b typically has a fastener 126 that extends through a lens mount slot 122c of lens mount 122 and into spectrometer base 104 or baseplate 104a. Between a head 126a of fastener 126 and lens mount 122 is a hold-down spring 128. There is sufficient spacing between lens mount slot 122c and fastener 126 to permit expansion/contraction of lens mount 122 caused by a temperature change. The coefficient of expansion of lens mount 122 is greater than the coefficient of expansion of spectrometer base 104 and/or baseplate 104a so that unfixed mount end 122b permits thermal expansion and contraction of thermal-compensating lens mount 122 in a direction shown by arrow 500, which is linear and transverse to the light beam from input slit 114. This structure allows achromatic lens 124 to slide relative to other components mounted on baseplate 104a and/or spectrometer base 104. Thermal-compensated lens mount 122 ensures that the plurality of light beams 138a, 138b, 138c will always impinge with sufficient intensity onto light-array detector 116 without affecting the electrical signal generated by light-array detector 116 notwithstanding a temperature change within spectrometer housing 102. One such material that meets the requirement that lens mount 122 have a greater coefficient of expansion than spectrometer base 104 and/or baseplate 104a (as the case may be) is a plastic that is a modified polyphenylene ether (PPE) resin consisting of amorphous blends of polyphenylene oxide (PPO) polyphenylene ether (PPE) resin and polystyrene sold under the trademark NORYL®.

Figure 18 illustrates an alternative embodiment of lens mount 122. In this embodiment, lens mount 122 has two fixed mount ends 122a, where each end 122a is secured to baseplate 104a and/or spectrometer base 104 by fastener 126. Because both ends 122a of lens mount 122 are fixed, any temperature change within spectrometer module 100 will affect angle of the plurality of light beams 138a, 138b, 138c and where they impinge on light-array detector 116. As previously disclosed regarding the slit image and the length of the light-array detector 116, a temperature change of greater than 0.5°C will cause the intensity of one of the light beams to not impinge completely on the light-array detector thereby causing an inaccurate reading. To nullify this potential effect, spectrometer module 100 is equipped with a temperature controller assembly (not shown) so that prism 131 and achromatic lens assembly 121 remain at a constant temperature. Although there are several methods available for maintaining the inside of spectrometer module 100 at a constant temperature, one example of such a temperature controller assembly to accomplish this is a ribbon heater with a thermistor (not shown) adhesively attached to the inside or outside of spectrometer module 100, which ribbon heater is controlled by an electronic regulation circuit (not shown). Optionally, spectrometer module 100 may also be insulated either inside or outside or both to more easily maintain a given temperature and protect against changes in temperature in the vicinity surrounding spectrometer module 100. Other mechanisms include placement of spectrometer module 100 within a temperature controlled environment.

Learning Data:

A data set of about 180 blood samples from approximately 15 different individuals was developed. The blood samples were manipulated using sodium nitrite to raise MetHb values, and using CO gas to raise COHb values. Plasma was removed from or added to samples to change the tHb level. Bilirubin spiking solution was added to vary the tBil level. A tonometer was used to manipulate the oxygen level. The blood samples were manipulated to cover a large range of analyte values. The blood samples were then measured on a reference lysing pHOx Ultra analyzer equipped with COOx analyzer and analysis software. The whole blood spectra were gathered on a pHOx Ultra analyzer equipped with the high-angle collection optics and other modifications of the present invention, as described earlier, with the lyse supply line completely disconnected and the whole blood samples running directly into the cuvette assembly 40 without lyse or any other dilution. Both analyzers were equipped with Zeonex windows in the respective cuvettes. This data set has been turned into a Matlab cell array file for use with Matlab scripts.

Prediction Model:

The next step in the calculation is to create a prediction model. Three models were developed for the analysis: one for the COOx parameters tHb and COHb, a second for HHb and MetHb, and a third for tBil. The quantity for O2Hb was determined by subtracting COHb, HHb, and MetHb from 100%. The X-data array was constructed from terms created from the measured absorbance at the wavelengths between 462-650 nm, 1 nm spacing. The tBil model was developed using the same set of data as the COOx model, except that samples with MetHb values greater than or equal to 20% were left out of the model. For each model, five Y-predictive values were assigned (O2Hb, HHb, COHb, MetHb, tBil) with tHb determined by adding the results for O2Hb, HHb, COHb, and MetHb. The number of Y-orthogonal values needed was determined by manual optimization of the correlation residual of the mapping function blood predictions with the reference analyzer values.

Using an initial calibration data set, the calibration sequence of a machine learning algorithm establishes a relationship between a matrix of known sample characteristics (the Y matrix) and a matrix of measured absorbance values at several wavelengths and potentially other measured values based on absorbance versus wavelength (the X matrix). Once this relationship is established, it is used by the analyzer to predict the unknown Y values from new measurements of X on whole blood samples.

Table 1 summarizes the settings and inputs used for the optimized models. The X-data consists of the absorbance and other terms based on absorbance vs. wavelength. In the process of optimizing the model, absorbance derivatives vs. wavelength were added. Models for analytes more sensitive to nonlinear scatter effects were built up with square root terms of the absorbance and its derivative. The model for analytes more affected by scatter had a correction term proportional to the fourth power of the wavelength. The X-vector row has one value for each wavelength for each of the three absorbance-based terms *f, g, and h* shown in the table for each model.

Table 1: Parameters used to construct algorithm models (KOPLS method).

| Model | Y-predictive components | Y-orthogonal components | X data structure (from absorbance vs. wavelength) | Kernel polynomial exponent |
|---|---|---|---|---|
| tHb, COHb | 5 | 4 | $f(\lambda) = \sqrt{\dfrac{dA(\lambda)}{d\lambda}}, g(\lambda) = \dfrac{dA(\lambda)}{d\lambda}, h(\lambda) = \sqrt{A(\lambda)}$ | 0.5 |
| HHb, MetHb | 5 | 4 | $f(\lambda) = \dfrac{dA(\lambda)}{d\lambda}, g(\lambda) = A(\lambda) \cdot \left(\dfrac{\lambda}{650nm}\right)^{4}, h(\lambda) = A(\lambda)$ | 1.0 |
| tBil | 5 | 16 | $f(\lambda) = \sqrt{\dfrac{dA(\lambda)}{d\lambda}}, g(\lambda) = \sqrt{A(\lambda)}, h(\lambda) = A(\lambda)$ | 1.0 |

The calibration set Y matrix is built up as follows from the known values of the calibration sample set of n lysed blood samples:

$$Y = \begin{bmatrix} tHb_1 & COHb_1 & HHHb_1 & MetHb_1 & tBil_1 \\ tHb_2 & COHb_2 & HHHb_2 & MetHb_2 & tBil_2 \\ \ldots & \ldots & \ldots & \ldots & \ldots \\ tHb_n & COHb_n & HHHb_n & MetHb_n & tBil_n \end{bmatrix}$$

where tHb is the total hemoglobin value of the lysed blood sample, COHb is the carboxyhemoblogin value of the lysed blood sample, HHb is the deoxyhemoglobin value of the lysed blood sample, MetHb is the methemoglobin value of the lysed blood sample, and tBil is the total bilirubin value of the lysed blood sample.

The X matrix is structured as follows:

$$X = \begin{bmatrix} f_1(\lambda_1), \ldots f_1(\lambda_n), & g_1(\lambda_1), \ldots g_1(\lambda_n), & h_1(\lambda_1), \ldots h_1(\lambda_n) \\ \vdots & \ddots & \vdots \\ f_n(\lambda_1), \ldots f_n(\lambda_n), & g_n(\lambda_1), \ldots g_n(\lambda_n), & h_n(\lambda_1), \ldots h_n(\lambda_n) \end{bmatrix}$$

where: $f,g,h$ are the absorbance-based functions listed in Table 1 versus wavelength, respectively.

The matrix X includes contributions from absorbance at the various wavelengths. The scope of the invention includes optionally adding other measurements to the calculation to reduce interferent effects.

Once these matrices are formed, they are used as the calibration set and the mapping function is computed according to the procedures particular to the machine learning algorithm chosen.

As described previously, conventional partial least squares, linear regression, linear algebra, neural networks, multivariate adaptive regression splines, projection to latent structures, kernel-based orthogonal projection to latent structures, or other machine learning mathematics is used with results obtained from the calibration set of data to determine the empirical relationship (or mapping function) between the absorbance values and the hemoglobin parameters. Typically, a mathematics package is used to generate the results where the package generally has options to select one of the machine learning mathematics known to those skilled in the art. Various mathematics packages exist and include, but are not limited to, Matlab by MatWorks of Natick, MA, "R" by R Project for Statistical Computing available over the Internet at www.r-project.org, Python from Python Software Foundation and available over the Internet at www.python.org in combination with Orange data mining software from Orange Bioinformatics available over the Internet at orange.biolab.si, to name a few.

It will be shown that the method of Kernel-Based Orthogonal Projection to Latent Structures (KOPLS) may be used as one type of machine learning algorithm to generate the mapping function. An explanation and description of KOPLS is best exemplified by the following references: Johan Trygg and Svante Wold. "Orthogonal projections to latent structures (O-PLS)." J. Chemometrics 2002; 16: 119-128; Mattias Rantalainen et al. "Kernel-based orthogonal projections to latent structures (K-OPLS)." J. Chemometrics 2007; 21: 376-385; and Max Bylesjö et al. "K-OPLS package: Kernel-based orthogonal projections to latent structures for prediction and interpretation in feature space." BMC Bioinformatics 2008, 9:106.

The kernel-based mathematics is useful in handling non-linear behavior in systems by using a kernel function to map the original data to a higher order space. Although any of the previously described machine learning mathematics may be used to enable one of ordinary skill in the art to practice the present invention, KOPLS has an additional advantage over other calculations such as, for example, conventional partial least squares because it can not only establish a relationship between quantified variations and analyte values to be determined, but can also remove unquantitated yet consistently present variation in the original data. These unquantitated variations might be due to analyzer and/or blood effects such as scatter losses and other interfering phenomena that are not explicitly measured. By extracting these unquantitated variations from the data, the method leaves behind in the data the information used to predict the measured values.

Using an initial training data set, the KOPLS model establishes a relationship (mapping function) between the matrix of known sample characteristics (the H matrix) , and a matrix of measured absorbance values at several wavelengths and potentially other measured values based on absorbance versus wavelength (the X matrix) as processed through a kernel function as specified by the KOPLS method. Once the KOPLS coefficients of this relationship are established, they are used with the kernel function by the analyzer to predict the unknown hemoglobin parameter values from new measurements of absorbance on samples.

The kernel function used in this example is a simple linear kernel function described in the Mattias Rantalainen et al. reference listed above and represented by the following equation:

$$\kappa(X, X) = \langle X, X \rangle$$

where the matrix of measured values X is put into the kernel function and subjected to further processing as specified in the cited KOPLS references above for creating the KOPLS training coefficients.

Once the set of training coefficients, or mapping function, is established, it is used to predict the hemoglobin parameter values and/or total bilirubin parameter values of a blood sample from future measurements. A single-row X matrix is created from the new measurements, then the value from this single-row X matrix is put through the kernel and mapping functions to produce the hemoglobin parameter values and/or total bilirubin parameter values according to the procedures necessary for the mapping function used according to the KOPLS procedures described in detail in the KOPLS references disclosed previously.

The data collected from the blood samples described above were put through the KOPLS method in a cross-validation process. Cross-validation is a process for using a data set to test a method. Several data rows are set aside and the rest are used to create a mapping function. The set-aside values are then used as "new" measurements and their Y matrix values calculated. This process is repeated by setting aside other measured values and computing another mapping function. By plotting the known values of the blood data vs. the calculated, the effectiveness of the method may be ascertained by inspecting the plot.

Turning now to Figures 18-23, there are illustrated graphical plots of the correlation results comparing the various hemoglobin parameters of lysed blood to whole blood using the KOPLS method. The blood samples were manipulated to cover a large range of analyte values. The technique of n-fold cross-validation using 60 folds was used to test the data. In this technique, the data set is divided into n=60 separate sets, and the model is made from n-1 of the sets, with the remaining set predicted using the model. The process is repeated 60 times for each group. Every data point is thus predicted using a model made from most of the other data points, without being included in the model.

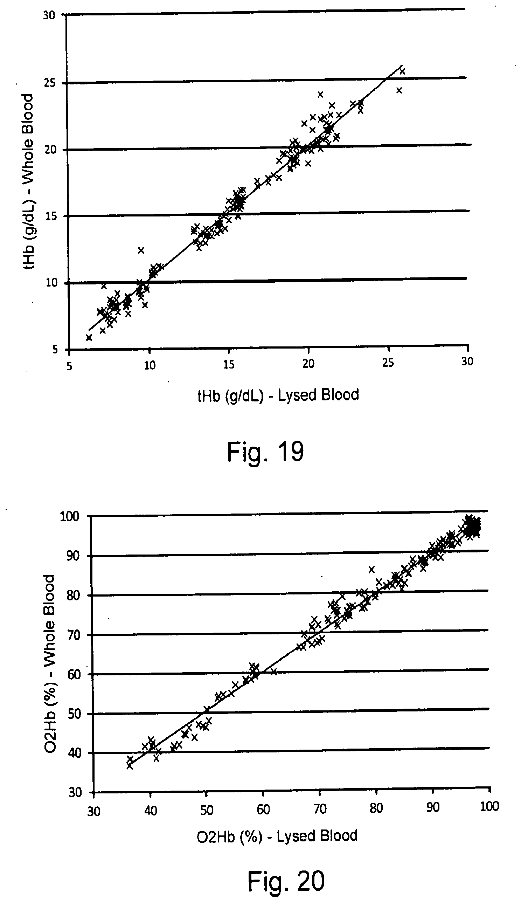

Fig. 19 shows the correlation results for tHb using the K-OPLS method. The horizontal axis has units representing the total hemoglobin in grams per deciliter of lysed blood. The vertical axis has units representing total hemoglobin in grams per deciliter of whole blood. As can be seen from the plot, the method of determining tHb of a whole blood sample has a correlation of greater than 99%.

Fig. 20 shows the correlation results for O2Hb using the K-OPLS method. The horizontal axis has units representing the percent oxyhemoglobin of lysed blood. The vertical axis has unit representing percent oxyhemoglobin of whole blood. As seen from the plot, the method of determining O2Hb of a whole blood sample has a correlation of greater than 99%.

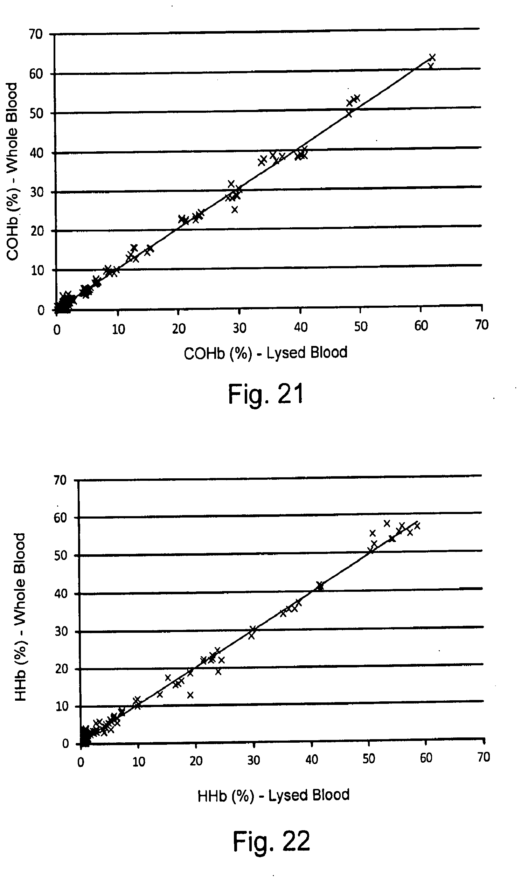

Fig. 21 shows the correlation results for carboxyhemoglobin using the K-OPLS method. The horizontal axis has units representing the percent carboxyhemoglobin of lysed blood. The vertical axis has unit representing percent carboxyhemoglobin of whole blood. As seen from the plot, the method of determining COHb of a whole blood sample has a correlation of greater than 99%.

Fig. 22 shows the correlation results for deoxyhemoglobin using the KOPLS method. The horizontal axis has units representing the percent deoxyhemoglobin of lysed blood. The vertical axis has unit representing percent deoxyhemoglobin of whole blood. As seen from the plot, the method of determining HHb of a whole blood sample has a correlation of greater than 99%.

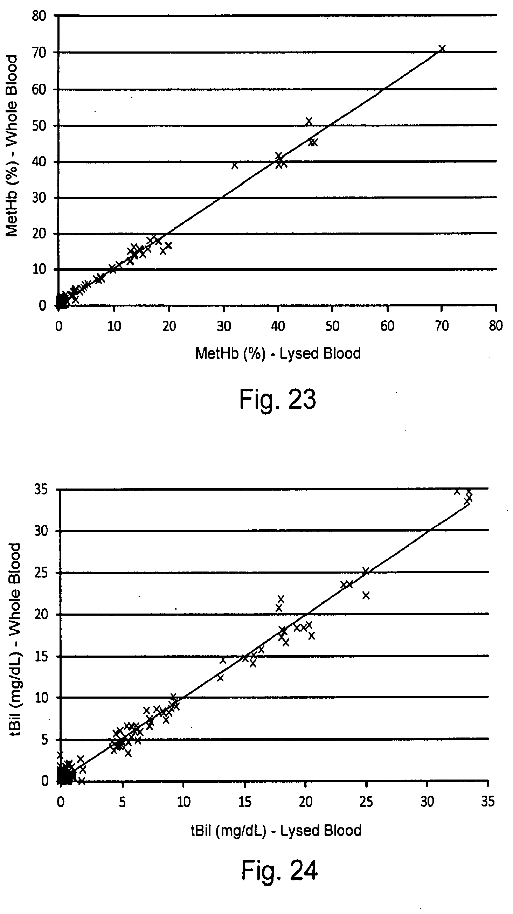

Fig. 23 shows the correlation results for methemoglobin using the KOPLS method. The horizontal axis has units representing the percent methemoglobin of lysed blood. The vertical axis has unit representing percent methemoglobin of whole blood. As seen from the plot, the method of determining MetHb of a whole blood sample has a correlation of greater than 99%.

Fig. 24 shows the correlation results for tBil using the K-OPLS method. The horizontal axis has units representing the total bilirubin in milligrams per deciliter of lysed blood. The vertical axis has units representing total bilirubin in milligrams per deciliter of whole blood. As can be seen from the plot, the method of determining tBil of a whole blood sample has a correlation of greater than 99%.

A method of making a whole blood measurement using the COOx analyzer subsystem 10 will now be described. An absorbance scan is measured by first recording a transmitted light intensity scan with cuvette module 43 filled with a transparent fluid such as water or analyzer flush solution otherwise known as the 'blank' scan. Then a transmitted light intensity scan with cuvette module 43 filled with the whole blood sample is recorded. After corrections for spectrometer dark response and detector linearity, the spectral absorbance is the negative of the logarithm to the base ten of the ratio of the whole blood scan to the transparent fluid scan computed at each wavelength in the measurement range.

More specifically, a depiction of the components of a COOx analyzer subsystem is shown in Figs. 1-18. This subsystem embodiment measures the optical absorbance of liquids introduced into cuvette module 43. The light used to perform the absorbance measurement originates from LED light source 28, is collected and transmitted by collimating lens 30, passes through first diffuser 32, circular polarizer 34, focusing lens 36, and optional protective window 38 before reaching cuvette module 43. Critical to an absolute absorbance measurement is knowledge of the cuvette path length. The cuvette path length is pre-measured for each individual cuvette module 43 and programmed into an electronic chip 48c on cuvette module 43. The path length information is read/retrieved by data processor module 130 of the analyzer whenever required.

After passing through cuvette module 43, the light is collected by lens 66, collimated and sent through second diffuser 68 and beam splitter 69. The purpose of beam splitter 69 is to allow light from calibrating light source 72 (for example, a krypton gas-discharge lamp), collimated by lens 74, to enter optical path 21. Calibrating light source 72 provides light at a few known wavelengths, which are used to periodically recalibrate the wavelength scale of spectrometer module 100. After passing through the beam splitter 69, the light is focused by lens 82 onto an optical fiber 92. The optical fiber 92 guides the light to input slit 114 of spectrometer module 100. The light passes through an achromatic lens 124, goes through light dispersion element 130 with a reflective back 132. The light is wavelength-dispersed by passing through light dispersion element 130 such as, for example, prism 130 then makes a return pass through the lens 124, which re-focuses the light onto the pixels of light-array detector 116. Light-array detector 116 converts the light energy into an electrical signal which represents the spectral intensity of the light. The electrical signal is sent to data processor module 150 for further processing and display of the final results to the user. Light-receiving and converting assembly 110 is a single board that holds input slit 114 and light-array detector 116 in close proximity as an integrated unit.

Input slit 114 is applied directly onto the same circuit board substrate 112 as and in close proximity to light-array detector 116. Other prior art spectrometers place these components on separate planes where they have separate mounting structures needing independent adjustment and alignment. The mounting scheme has several advantages that lower the cost and size of spectrometer module 100: 1) cost of separate mounting structures is avoided, 2) input slit 114 can be laser etched in a precise position relative to light-array detector 116 making alignment less labor intensive, 3) inexpensive spherical surface optics can be used in the optical system since the image of the slit on the detector is only slightly off-axis from the center axis of the optical system, minimizing aberration, and 4) a single alignment procedure for a unified slit and detector assembly replaces alignment procedures for two separate assemblies.

It is important to note that first diffuser 32 and second diffuser 68 are positioned before and after cuvette module 43, respectively. Optical absorbance measurement of a diffuse sample presents a unique problem. The diffuse transmittance of the sample scrambles the initial spatial light distribution of the measurement system caused by the nonuniformity typical of light sources. Thus, the spatial light distribution of the 'blank' scan can be quite different from the whole blood sample scan. Since optical detectors have response that varies spatially, the response can vary due to spatial distribution changes of the incident light, even if the overall intensity has not changed. An absorbance scan which is based on the ratio of the sample scan to the blank scan will have a significant absorbance component due to this effect in addition to the absorbance due to the sample alone. This results in a significant measurement error of the sample absorbance that is intolerable for cooximetry.

The advantage of placing cuvette module 43 between first and second diffusers 32, 68 is that the spatial light distribution will appear the same for the blank and sample scans, removing this error effect. Diffusers 32, 68 are specially chosen so that they diffuse a ray of incident light into the full acceptance cone of the optical system, but not more so, so that as much light throughput as possible may be preserved while scrambling the light ray completely across the field.

Although the preferred embodiments of the present invention have been described herein, the above description is merely illustrative. Further modification of the invention herein disclosed will occur to those skilled in the respective arts and all such modifications are deemed to be within the scope of the invention as defined by the appended claims.

## Claims

**1.** An optical absorbance measurement system (10) for measuring whole-blood hemoglobin parameters comprising:

an optical-sample module (20) comprising:

a light-emitting module (22) having an LED light source (28) capable of emitting light wherein the light is directed thereby defining an optical path (21);
a replaceable cuvette assembly (40) adjacent the light-emitting module (22), wherein the cuvette assembly (40) is adapted for receiving a whole-blood sample and includes a cuvette substrate (41) and a cuvette module (43) on the cuvette substrate (41), the cuvette module (43) including a cuvette first portion (44) and a cuvette second portion (52) bonded together, the cuvette first portion (41) having a sample receiving recess (45), a first cuvette window (49) and an electronic chip assembly (48) and the cuvette second portion (52) having a second cuvette window (52), wherein the sample receiving recess (45) of the cuvette first portion (44) and the cuvette second portion (52) form a sample receiving chamber (54) with the first cuvette window (49) and the second cuvette window (52) aligned with the first cuvette window (49), the sample receiving chamber (54) disposed in the optical path (21) for receiving light from the LED light source (28), the distance between the first cuvette window (49) and the second cuvette window (52) defining an optical path length (43a) of the sample receiving chamber (54), and wherein an electronic chip (48c) of the electronic chip assembly (48) comprises the optical path length (43a) of the sample receiving chamber (54) pre-measured and programmed into the electronic chip (48c);

a first optical diffuser (32) positioned within the optical path (21) between the LED light source (28) and the replaceable cuvette assembly (40);

a calibrating-light module (60) after the replaceable cuvette module (40) having a beam splitter (69) and a calibrating-light source (72) with one or more known wavelengths of light, the calibrating-light module (60) capable of emitting a calibrating light (72a) transverse to the optical path (21) into the optical path (21) at the beam splitter (69); and

a second optical diffuser (68) positioned within the optical path (21) downstream from the replaceable cuvette assembly (40) and upstream from the beam splitter (69);

an optical fiber (92) having a light-receiving end (92a) and a light-emitting end (92b), the light-receiving end (92a) optically connected to the calibrating-light module (60) downstream from the beam splitter (69) wherein the light-receiving end (92a) receives the light emitted along the optical path (21) and conducts the light to the light-emitting end (92b);

a spectrometer module (100) capable of receiving the light from the light-emitting end (92b) of the optical fiber (92), separating the light into a plurality of light beams wherein each light beam has a different wavelength, and converting the plurality of light beams into an electrical signal; and

a processor module (150) capable of

(1) obtaining the pre-measured optical path length of the sample receiving chamber (54) from the electronic chip (48c) and

(2) receiving and processing the electrical signal from the spectrometer module (100) generated for a whole-blood sample and, with the obtained optical path length of the sample receiving chamber (54), transforming the electrical signal into an output signal useable for displaying and reporting hemoglobin parameter values and/or total bilirubin parameter values for the whole-blood sample.

2. The optical absorbance measurement system (10) of Claim 1 wherein the light-emitting module (22) includes a plurality of optical components (B) disposed in the optical path (21) between the LED light source (28) and the cuvette assembly (40), the plurality of optical components (B) includes at least the first optical diffuser (32) and one or more of a collimating lens (30), a circular polarizer (34), and a focusing lens (36).

3. The optical absorbance measurement system (10) of Claim 1 wherein the spectrometer module (100) comprising:

an input slit (114) positioned in the optical path (21) to receive the light emitted from the light-emitting end (92b) of the optical fiber (92) and to transmit the light therethrough;

an optical component group (120) having an achromatic lens (124) and a light dispersing element (130) wherein the light dispersing element (130) is disposed in the optical path (21) wherein the light dispersing element (130) is capable of receiving the light transmitted through the input slit (114) and the achromatic lens (124), separating the light into the plurality of light beams wherein each light beam has a different wavelength, and re-directing the plurality of light beams back through the achromatic lens (124) toward but offset from the input slit (114); and

a light-array detector (116) capable of receiving the plurality of light beams and converting the plurality of light beams into an electrical signal.

4. A method of measuring whole-blood hemoglobin parameters despite strong optical scattering caused by whole blood, the method comprising:

providing a LED light source (28) with a spectral range of about 422 nm to about 695 nm;

guiding light having the spectral range from the LED light source (28) along an optical path (21);

providing a replaceable cuvette module (43) including a cuvette substrate (41) and a cuvette module (43) on the cuvette substrate (41) by bonding together a cuvette first portion (44) and a cuvette second portion (52), the cuvette first portion (41) having a sample receiving recess (45), a first cuvette window (49) and an electronic chip assembly (48) and the cuvette second portion (52) having a second cuvette window (52), wherein the sample receiving recess (45) of the cuvette first portion (44) and the cuvette second portion (52) form a sample receiving chamber (54) with the first cuvette window (49) and the second cuvette window (52) aligned with the first cuvette window (49) disposed in the optical path (21), the distance between the first cuvette window (49) and the second cuvette window (52) defining an optical path length of the sample receiving chamber (54) that is pre-measured and stored on an electronic chip (48c) of the electronic chip assembly (48) of the cuvette module (43), wherein the second cuvette window (52) of the cuvette module (43) transmits the light through the sample receiving chamber (54) and through the first cuvette window (49) wherein the sample receiving chamber (54) contains a sample of whole blood;

providing a pair of first and second optical diffusers (32, 68) disposed in the optical path (21), wherein the first cuvette window (49) and the second cuvette window (52) of the sample receiving chamber (54) of the cuvette module (43) are disposed between the pair of first and second optical diffusers (32, 68);

providing a calibrating light source (72) after the replaceable cuvette module (43) capable of emitting a calibrating light beam (72a) into the optical path (21) at a beam splitter (69) transverse to the optical path (21);

guiding light from the optical path (21) after the second diffusor (68) into a spectrometer (100) having a light dispersing element (130) that separates the light into a plurality of light beams wherein each light beam has a different wavelength and converts the plurality of light beams into an electrical signal;

obtaining the pre-measured and stored optical path length of the sample receiving chamber (54) from the electronic chip (48c); and

processing the electrical signal from the spectrometer module (100) generated for the whole-blood sample and, with the obtained optical path length of the sample receiving chamber (54), transforming the electrical signal into an output signal useable for displaying and reporting hemoglobin parameter values and/or total bilirubin parameter values of the sample of whole blood.

5. The method of Claim 4 wherein the processing step further includes processing the electrical signal to spectral absorbance and then mapping the spectral absorbance to hemoglobin parameter values and/or bilirubin parameter values using a computational mapping function, and, includes using a kernel-based orthogonal projection to latent structures mapping function as the computational mapping function.

**Patentansprüche**

1. System zur optischen Extinktionsmessung (10) zum Messen von Vollblut-Hämoglobinparametern, umfassend:

ein optisches Probenmodul (20) umfassend:

ein lichtemittierendes Modul (22) mit einer LED-Lichtquelle (28), die Licht emittieren kann, wobei das Licht gerichtet ist, wodurch ein Strahlengang (21) definiert wird;
eine austauschbare Küvettenanordnung (40) neben dem lichtemittierenden Modul (22), wobei die Küvettenanordnung (40) zum Aufnehmen einer Vollblutprobe ausgelegt ist und ein Küvettensubstrat (41) und ein Küvettenmodul (43) auf dem Küvettensubstrat (41) umfasst, wobei das Küvettenmodul (43) einen ersten Küvettenabschnitt (44) und einen zweiten Küvettenabschnitt (52) umfasst, die miteinander gebondet sind, wobei der erste Küvettenabschnitt (41) eine Probenaufnahmevertiefung (45), ein erstes Küvettenfenster (49) und eine elektronische Chipanordnung (48) aufweist und der zweite Küvettenabschnitt (52) ein zweites Küvettenfenster (52) aufweist, wobei die Probenaufnahmevertiefung (45) des ersten Küvettenabschnitts (44) und der zweite Küvettenabschnitt (52) eine Probenaufnahmekammer (54) mit dem ersten Küvettenfenster (49) und dem zweiten Küvettenfenster (52), das mit dem ersten Küvettenfenster (49) ausgerichtet ist, bilden, wobei die Probenaufnahmekammer (54) im Strahlengang (21) zum Empfangen von Licht von der LED-Lichtquelle (28) angeordnet ist, der Abstand zwischen dem ersten Küvettenfenster (49) und dem zweiten Küvettenfenster (52) eine optische Weglänge (43a) der Probenaufnahmekammer (54) definieren und
wobei ein elektronischer Chip (48c) der elektronischen Chipanordnung (48) die optische Weglänge (43a) der Probenaufnahmekammer (54) umfasst, die vorab gemessen und in den elektronischen Chip (48c) programmiert ist;
einen ersten optischen Diffusor (32), der innerhalb des Strahlengangs (21) zwischen der LED-Lichtquelle (28) und der austauschbaren Küvettenanordnung (40) angeordnet ist;
ein Kalibrierlichtmodul (60) nach dem austauschbaren Küvettenmodul (40) mit einem Strahlteiler (69) und einer Kalibrierlichtquelle (72) mit einer oder mehreren bekannten Lichtwellenlängen, wobei das Kalibrierlichtmodul (60) fähig ist, ein Kalibrierlicht (72a) quer zum Strahlengang (21) in den Strahlengang (21) am Strahlteiler (69) zu emittieren; und

einen zweiten optischen Diffusor (68), der innerhalb des Strahlengangs (21) stromabwärts von der austauschbaren Küvettenanordnung (40) und stromaufwärts von dem Strahlteiler (69) positioniert ist;

eine optische Faser (92) mit einem lichtempfangenden Ende (92a) und einem lichtemittierenden Ende (92b), wobei das lichtempfangende Ende (92a) optisch mit dem Kalibrierlichtmodul (60) stromabwärts von dem Strahlteiler (69) verbunden ist,

wobei das lichtempfangende Ende (92a) das entlang des Strahlengangs (21) emittierte Licht empfängt und das Licht zum lichtemittierenden Ende (92b) leitet;

ein Spektrometermodul (100), das fähig ist, das Licht von dem lichtemittierenden Ende (92b) der optischen Faser (92) zu empfangen, das Licht in eine Mehrzahl von Lichtstrahlen aufzuteilen, wobei jeder Lichtstrahl eine unterschiedliche Wellenlänge hat, und die Mehrzahl von Lichtstrahlen in ein elektrisches Signal umzuwandeln; und

ein Prozessormodul (150), das fähig ist zum

(1) Erhalten der vorab gemessenen optischen Weglänge der Probenaufnahmekammer (54) von dem elektronischen Chip (48c) und

(2) Empfangen und Verarbeiten des für eine Vollblutprobe erzeugten elektrischen Signals von dem Spektrometermodul (100) und Umwandeln des elektrischen Signals mit der erhaltenen optischen Weglänge der Probenaufnahmekammer (54) in ein Ausgangssignal, das zum Anzeigen und Melden von Hämoglobinparameterwerten und/oder Gesamtbilirubinparameterwerten für die Vollblutprobe verwendbar ist.

2. System zur optischen Extinktionsmessung (10) nach Anspruch 1, wobei das lichtemittierende Modul (22) eine Mehrzahl optischer Komponenten (B) umfasst, die in dem Strahlengang (21) zwischen der LED-Lichtquelle (28) und der Küvettenanordnung (40) angeordnet sind, wobei die Mehrzahl optischer Komponenten (B) zumindest den ersten optischen Diffusor (32) und eine oder mehrere von einer Kollimatorlinse (30), einem Zirkularpolarisator (34) und einer Fokussierlinse (36) umfasst.

3. System zur optischen Extinktionsmessung (10) nach Anspruch 1, wobei das Spektrometermodul (100) umfasst:

einen Eingangsschlitz (114), der in dem Strahlengang (21) positioniert ist, um das von dem lichtemittierenden Ende (92b) der optischen Faser (92) emittierte Licht zu empfangen und um das Licht dort hindurch zu übertragen; eine optische Komponentengruppe (120) mit einer achromatischen Linse (124) und einem lichtstreuenden Element (130), wobei das lichtstreuende Element (130) in dem Strahlengang (21) angeordnet ist, wobei das lichtstreuende Element (130) fähig ist zum Empfangen des durch den Eingangsschlitz (114) und die achromatische Linse (124) übertragenen Lichts, Aufteilen des Lichts in die Mehrzahl von Lichtstrahlen, wobei jeder Lichtstrahl eine unterschiedliche Wellenlänge hat, und Umlenken der Mehrzahl von Lichtstrahlen zurück durch die achromatische Linse (124) zum Eingangsschlitz (114), aber versetzt davon; und einen Lichtfelddetektor (116), der fähig ist zum Empfangen der Mehrzahl von Lichtstrahlen und Umwandeln der Mehrzahl von Lichtstrahlen in ein elektrisches Signal.

4. Verfahren zur Messung von Vollblut-Hämoglobinparametern trotz starker optischer Streuung, die durch Vollblut verursacht wird, wobei das Verfahren umfasst:

Bereitstellen einer LED-Lichtquelle (28) mit einem Spektralbereich von etwa 422 nm bis etwa 695 nm; Leiten von Licht mit dem Spektralbereich von der LED-Lichtquelle (28) entlang eines Strahlengangs (21); Bereitstellen eines austauschbaren Küvettenmoduls (43) mit einem Küvettensubstrat (41) und einem Küvettenmodul (43) auf dem Küvettensubstrat (41) durch Bonden eines ersten Küvettenabschnitts (44) und eines zweiten Küvettenabschnitts (52), wobei der erste Küvettenabschnitt (41) eine Probenaufnahmevertiefung (45), ein erstes Küvettenfenster (49) und eine elektronische Chipanordnung (48) aufweist und der zweite Küvettenabschnitt (52) ein zweites Küvettenfenster (52) aufweist, wobei die Probeaufnahmevertiefung (45) des ersten Küvettenabschnitts (44) und des zweiten Küvettenabschnitts (52) eine Probenaufnahmekammer (54) mit dem ersten Küvettenfenster (49) und dem zweiten Küvettenfenster (52) bilden, das mit dem ersten Küvettenfenster (49) ausgerichtet ist, das im Strahlengang (21) angeordnet ist, wobei der Abstand zwischen dem ersten Küvettenfenster (49) und dem zweiten Küvettenfenster (52) eine optische Weglänge der Probenaufnahmekammer (54) definiert, die vorab gemessen und auf einem elektronischen Chip (48c) der elektronischen Chipanordnung (48) des Küvettenmoduls (43) gespeichert ist, wobei das zweite Küvettenfenster (52) des Küvettenmoduls (43) das Licht durch die Probenaufnahmekammer (54) und durch das erste Küvettenfenster (49) überträgt, wobei die Probenaufnahmekammer (54) eine Vollblutprobe enthält;

Bereitstellen eines Paars erster und zweiter optischer Diffusoren (32, 68), die im Strahlengang (21) angeordnet sind, wobei das erste Küvettenfenster (49) und das zweite Küvettenfenster (52) der Probenaufnahmekammer (54) des Küvettenmoduls (43) zwischen dem Paar erster und zweiter optischer Diffusoren (32, 68) angeordnet sind;

Bereitstellen einer Kalibrierlichtquelle (72) nach dem austauschbaren Küvettenmodul (43), die fähig ist, einen Kalibrierlichtstrahl (72a) in den Strahlengang (21) an einem Strahlteiler (69) quer zum Strahlengang (21) zu emittieren;

Leiten von Licht aus dem Strahlengang (21) nach dem zweiten Diffusor (68) in ein Spektrometer (100) mit einem lichtstreuenden Element (130), das das Licht in eine Mehrzahl von Lichtstrahlen trennt, wobei jeder Lichtstrahl eine unterschiedliche Wellenlänge hat, und die Mehrzahl von Lichtstrahlen in ein elektrisches Signal umwandelt;

Erhalten der vorab gemessenen und gespeicherten optischen Weglänge der Probenaufnahmekammer (54) von dem elektronischen Chip (48c); und

Verarbeiten des für die Vollblutprobe erzeugten elektrischen Signals von dem Spektrometermodul (100) und Umwandeln des elektrischen Signals mit der erhaltenen optischen Weglänge der Probenaufnahmekammer (54) in ein Ausgangssignal, das zum Anzeigen und Melden von Hämoglobinparameterwerten und/oder Gesamtbilirubinparameterwerten der Vollblutprobe verwendbar ist.

5. Verfahren nach Anspruch 4, wobei der Verarbeitungsschritt ferner ein Verarbeiten des elektrischen Signals zu spektraler Extinktion und anschließendes Abbilden der spektralen Extinktion auf Hämoglobinparameterwerte und/oder Bilirubinparameterwerte unter Verwendung einer rechnerischen Abbildungsfunktion umfasst und ein Verwenden einer Abbildungsfunktion einer kernbasierten orthogonalen Projektion auf latente Strukturen als die rechnerische Abbildungsfunktion umfasst.

## Revendications

1. Système de mesure d'absorbance optique (10) pour mesurer des paramètres d'hémoglobine du sang total comprenant :

un module d'échantillonnage optique (20) comprenant :

un module électroluminescent (22) ayant une source de lumière à DEL (28) apte à émettre de la lumière dans laquelle la lumière est dirigée, définissant ainsi un trajet optique (21) ;

un ensemble cuvette remplaçable (40) adjacent au module électroluminescent (22), dans lequel l'ensemble cuvette (40) est adapté pour recevoir un échantillon de sang total et comprend un substrat de cuvette (41) et un module de cuvette (4, 3) sur le substrat de cuvette (41), le module de cuvette (43) comprenant une première partie de cuvette (44) et une seconde partie de cuvette (52) liées ensemble, la première partie de cuvette (41) ayant un creux de réception d'échantillon (45), une premier fenêtre de cuvette (49) et un ensemble puce électronique (48) et la seconde partie de cuvette (52) ayant une seconde fenêtre de cuvette (52), dans lequel le creux de réception d'échantillon (45) de la première partie de cuvette (44) et la seconde partie de cuvette partie (52) forment une chambre de réception d'échantillon (54) avec la première fenêtre de cuvette (49) et la seconde fenêtre de cuvette (52) alignée avec la première fenêtre de cuvette (49), la chambre de réception d'échantillon (54) étant disposée dans le trajet optique (21) pour recevoir la lumière de la source de lumière à DEL (28), la distance entre la première fenêtre de cuvette (49) et la seconde fenêtre de cuvette (52) définissant une longueur de trajet optique (43a) de la chambre de réception d'échantillon (54), et dans lequel une puce électronique (48c) de l'ensemble puce électronique (48) comprend la longueur de trajet optique (43a) de la chambre de réception d'échantillon (54) pré-mesurée et programmée dans la puce électronique (48c) ;

un premier diffuseur optique (32) positionné à l'intérieur du trajet optique (21) entre la source de lumière à DEL (28) et l'ensemble cuvette remplaçable (40) ;

un module de lumière d'étalonnage (60) après le module de cuvette remplaçable (40) ayant un séparateur de faisceau (69) et une source de lumière d'étalonnage (72) avec une ou plusieurs longueurs d'onde de lumière connues, le module de lumière d'étalonnage (60) étant apte à émettre une lumière d'étalonnage (72a) transversalement au trajet optique (21) dans le trajet optique (21) au niveau du séparateur de faisceau (69) ; et

un second diffuseur optique (68) positionné à l'intérieur du trajet optique (21) en aval de l'ensemble cuvette remplaçable (40) et en amont du séparateur de faisceau (69) ;

une fibre optique (92) ayant une extrémité de réception de lumière (92a) et une extrémité d'émission de lumière (92b), l'extrémité de réception de lumière (92a) étant connectée optiquement au module de lumière d'étalonnage (60) en aval du séparateur de faisceau (69) dans laquelle l'extrémité de réception de lumière (92a) reçoit la lumière émise le long du trajet optique (21) et conduit la lumière vers l'extrémité d'émission de lumière (92b) ;

un module spectromètre (100) apte à recevoir la lumière de l'extrémité d'émission de lumière (92b) de la fibre optique (92), à séparer la lumière en une pluralité de faisceaux lumineux, chaque faisceau lumineux ayant une longueur d'onde différente, et à convertir la pluralité de faisceaux lumineux en un signal électrique ; et

un module processeur (150) apte à

(1) obtenir la longueur de trajet optique pré-mesurée de la chambre de réception d'échantillon (54) à partir de la puce électronique (48c) et

(2) recevoir et à traiter le signal électrique provenant du module spectromètre (100) généré pour un échantillon de sang total et, avec la longueur de trajet optique obtenue de la chambre de réception d'échantillon (54), à transformer le signal électrique en un signal de sortie utilisable pour afficher et rapporter des valeurs de paramètres d'hémoglobine et/ou des valeurs de paramètres de bilirubine totale pour l'échantillon de sang total.

2.  Système de mesure d'absorbance optique (10) selon la revendication 1, dans lequel le module électroluminescent (22) comprend une pluralité de composants optiques (B) disposés dans le trajet optique (21) entre la source de lumière à DEL (28) et l'ensemble cuvette (40), la pluralité de composants optiques (B) comprend au moins le premier diffuseur optique (32) et un ou plusieurs éléments parmi une lentille de collimation (30), un polariseur circulaire (34) et une lentille de focalisation (36).

3.  Système de mesure d'absorbance optique (10) selon la revendication 1, dans lequel le module spectromètre (100) comprend :

une fente d'entrée (114) positionnée dans le trajet optique (21) pour recevoir la lumière émise à partir de l'extrémité d'émission de lumière (92b) de la fibre optique (92) et pour transmettre la lumière à travers cette dernière ;

un groupe de composants optiques (120) ayant une lentille achromatique (124) et un élément de dispersion de lumière (130) dans lequel l'élément de dispersion de lumière (130) est disposé dans le trajet optique (21) dans lequel l'élément de dispersion de lumière (130) est apte à recevoir la lumière transmise à travers la fente d'entrée (114) et la lentille achromatique (124), à séparer la lumière en la pluralité de faisceaux lumineux, chaque faisceau lumineux ayant une longueur d'onde différente, et à rediriger la pluralité de faisceaux lumineux à travers la lentille achromatique (124) vers la fente d'entrée (114) mais décalés de cette dernière ; et

un détecteur à réseau lumineux (116) apte à recevoir la pluralité de faisceaux lumineux et à convertir la pluralité de faisceaux lumineux en un signal électrique.

4.  Procédé de mesure de paramètres d'hémoglobine du sang total malgré une forte diffusion optique provoquée par le sang total, le procédé comprenant :

la fourniture d'une source de lumière à DEL (28) avec une plage spectrale d'environ 422 nm à environ 695 nm ;

le guidage de la lumière ayant la plage spectrale à partir de la source de lumière à DEL (28) le long d'un trajet optique (21) ;

la fourniture d'un module de cuvette remplaçable (43) comprenant un substrat de cuvette (41) et un module de cuvette (43) sur le substrat de cuvette (41) par liaison ensemble d'une première partie de cuvette (44) et d'une seconde partie de cuvette (52), la première partie de cuvette (41) ayant un creux de réception d'échantillon (45), une première fenêtre de cuvette (49) et un ensemble puce électronique (48) et la seconde partie de cuvette (52) ayant une seconde fenêtre de cuvette (52), dans lequel le creux de réception d'échantillon (45) de la première partie de cuvette (44) et la seconde partie de cuvette (52) forment une chambre de réception d'échantillon (54) avec la première fenêtre de cuvette (49) et la seconde fenêtre de cuvette (52) alignée avec la première fenêtre de cuvette (49) est disposée dans le trajet optique (21), la distance entre la première fenêtre de cuvette (49) et la seconde fenêtre de cuvette (52) définissant une longueur de trajet optique de la chambre de réception d'échantillon (54) qui est pré-mesurée et stockée sur une puce électronique (48c) de l'ensemble puce électronique (48) du module de cuvette (43), dans lequel la seconde fenêtre de cuvette (52) du module de cuvette (43) transmet la lumière à travers la chambre de réception d'échantillon (54) et à travers la première fenêtre de cuvette (49) dans lequel la chambre de réception d'échantillon (54) contient un échantillon de sang total ;

la fourniture d'une paire de premier et second diffuseurs optiques (32, 68) disposés dans le trajet optique (21), la première fenêtre de cuvette (49) et la seconde fenêtre de cuvette (52) de la chambre de réception d'échantillon (54) du module de cuvette (43) étant disposées entre la paire de premier et second diffuseurs optiques (32, 68) ;

la fourniture d'une source de lumière d'étalonnage (72) après le module de cuvette remplaçable (43) apte à émettre un faisceau lumineux d'étalonnage (72a) dans le trajet optique (21) au niveau d'un séparateur de faisceau (69) transversal au trajet optique (21) ;

le guidage de la lumière du trajet optique (21) après le second diffuseur (68) dans un spectromètre (100) ayant un élément de dispersion de lumière (130) qui sépare la lumière en une pluralité de faisceaux lumineux, chaque faisceau lumineux ayant une longueur d'onde différente et qui convertit la pluralité de faisceaux lumineux en un signal électrique ;

l'obtention de la longueur du trajet optique pré-mesurée et stockée de la chambre de réception d'échantillon (54) à partir de la puce électronique (48c) ; et

le traitement du signal électrique provenant du module spectromètre (100) généré pour l'échantillon de sang total et, avec la longueur de trajet optique obtenue de la chambre de réception d'échantillon (54), la transformation du signal électrique en un signal de sortie utilisable pour afficher et rapporter des valeurs de paramètre d'hémoglobine et/ou des valeurs de paramètres de bilirubine totale de l'échantillon de sang total.

**5.** Procédé selon la revendication 4, dans lequel l'étape de traitement comprend en outre le traitement du signal électrique en absorbance spectrale, puis le mappage de l'absorbance spectrale sur les valeurs de paramètres d'hémoglobine et/ou les valeurs de paramètres de bilirubine à l'aide d'une fonction de mappage informatique, et, comprend l'utilisation d'une fonction de mappage de projection orthogonale basée sur le noyau sur des structures latentes en tant que fonction de mappage informatique.

Fig. 1

Fig. 2

Fig. 3

Fig. 3A

Fig. 3B

**Fig. 4**

**Fig. 5**

**Fig. 6**

44

47

46

45

49

48a

**Fig. 7**

**Fig. 8**

47

48b

48c

49

45

Fig. 9

Fig. 10

Fig. 11

Fig. 12

Fig. 13

Fig. 14

Fig. 15

Fig. 16

165

100

## Fig. 17A

160

170

100

## Fig. 17B

Fig. 17C

Fig. 18

Fig. 19

Fig. 20

Fig. 21

Fig. 22

Fig. 23

Fig. 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150316471 A1

**Non-patent literature cited in the description**

- **JOHAN TRYGG ; SVANTE WOLD.** Orthogonal projections to latent structures (O-PLS). *J. Chemometrics,* 2002, vol. 16, 119-128
- **MATTIAS RANTALAINEN et al.** Kernel-based orthogonal projections to latent structures (K-OPLS). *J. Chemometrics,* 2007, vol. 21, 376-385
- **MAX BYLESJÖ et al.** K-OPLS package: Kernel-based orthogonal projections to latent structures for prediction and interpretation in feature space. *BMC Bioinformatics,* 2008, vol. 9, 106